# EUROPEAN PATENT APPLICATION

(11) **EP 4 628 215 A1**
(43) Date of publication of application: **08.10.2025**
(21) Application number: 24200324.2
(22) Date of filing: 13.09.2024
(51) Int. Cl.: B01L 3/00

(54) **TEST DEVICE**

(30) Priority: 02.04.2024 CN 202410398061
(71) Applicant: Zhejiang Orient Gene Biotech Co., Ltd, Anji Huzhou Zhejiang 313300 (CN)
(72) Inventor: Shen, Lili, Huzhou, Zhejiang, 313300 (CN); Lei, Siyu, Huzhou, Zhejiang, 313300 (CN); Fang, Jianqiu, Huzhou, Zhejiang, 313300 (CN)
(74) Representative: Petraz, Gilberto Luigi

(57) **Abstract**

The invention provides a device, and the device includes: a sample chamber for receiving a sample collector, where the sample chamber is provided with an easy-to-pierce sealing film; and an accommodating chamber for receiving the sample chamber, where the accommodating chamber includes a piercing element for piercing the sealing film to release a liquid in the sample chamber therein, and a blade structure for laterally cutting the sealing film.

## Description

### CROSS-REFERENCE TO RELATED APPLICATIONS

This application claims priority to Chinese Patent Application, Application No.: 2024103980611, filed on April 2, 2024, and all disclosures of this application are incorporated by reference in their entirety as a part of this application.

### BACKGROUND OF THE INVENTION

### Field of the Invention

The invention relates to a device for collecting and testing a liquid sample, and in particular, to a device for testing an analyte in a liquid sample in the field of rapid diagnosis, such as collection of urine and saliva and testing analytes in samples.

### Description of the Related Art

The following description is merely an introduction of some background knowledge and does not constitute any limitation to the invention.

In the field of in vitro diagnosis (IVD), chromatographic techniques are often used for diagnosing and detecting diseases and other items. For example, immune colloidal gold test strip, dry chemical test strip, immunofluorescence test strip, and the like all react with reagents after samples are pretreated based on the chromatographic theory, so as to finally obtain diagnosis results reflecting whether patients suffer from diseases. The function process of the immunofluorescence test strip is that: after samples (whole blood, plasma, and the like) are dripped into a sample application pad, liquid flows to an absorbent filter paper; the samples are treated in the sample application pad to filtrate erythrocytes and remove interfering substances and the like; when flowing through a conjugate pad, the samples immunobind with antigens and antibodies and carry fluorophores; when flowing through a nitrocellulose membrane, the samples specifically bind with antigens and antibodies bound thereon in advance; and fluorophores gathered on a testing line and a control line can reflect test results, and other interfering substances unbound are absorbed by the absorbent filter paper. Fluorescence immunochromatography has been widely used in the field of POCT detection in recent years because of its simple operation, strong specificity, high sensitivity, and quantification. However, in recent decades, most of immunochromatographic test cards can be used for detection of a single item only in a form of a single card with a single test strip. However, with the development of medical technologies, multiple targets need to be detected at the same time during diagnosis of diseases for more accurate determination, such as myocardial 3-item joint examination and myocardial 5-item joint examination. Under some circumstances, it is necessary to detect the status of multiple organs at the same time to determine the diseases, such as cardiopulmonary 5-item joint examination.

At present, the testing device for detecting the presence or absence of an analyte in sample is widely used in hospitals or homes, and these testing devices for rapid diagnosis include one or more test strips, such as early pregnancy detection and drug abuse detection. Such testing devices for rapid diagnosis are very convenient, and can obtain testing results from the test strips in one minute or at most ten minutes or so. Drug tests are widely used by the drug control department, the Public Security Bureau, drug rehabilitation centers, physical examination centers, physical examination offices of national conscription, etc. Drug tests are diverse and frequent. In some cases, samples need to be collected and then tested by professional testing agencies or test laboratories. Some tests need to be completed in the site in time, for example, roadsides, for example, persons who drive after drug use need to be tested on the spot (referred to as "drug driving"), to obtain the testing results in time.

Conventionally, for fecal testing, feces are collected by a collector, and analytes in the samples are tested. Some of the samples are collected and stored by special collection tools, and then sent to professional institutions for testing. Such operation is inconvenient, and during transportation of the samples, the samples will deteriorate and affect the final test and testing results. At present, although there are some integrated fecal collection and testing structures, they always have some disadvantages, such as the control of the sample size collected and the failure of sampling for some special samples. In particular, such structures are very convenient for home self-testing operation. Sometimes, when analytes in feces are tested, it is desired that they can be tested after being collected to obtain the testing results, or test subjects can detect them at home, which requires improving the existing conventional sample collection and detection.

### BRIEF SUMMARY OF THE INVENTION

In order to overcome disadvantages in the prior art, the invention provides a device for testing an analyte in a liquid sample, and the device includes a sample chamber for receiving a collector and an accommodating chamber for receiving the sample cavity; therefore, when the sample chamber enters the accommodating chamber, the liquid sample is released from the sample chamber and used for test and assay.

In some embodiments, the sample chamber for receiving a collector includes a treatment liquid for treating the liquid sample; and when the sample chamber is inserted into the accommodating chamber, the sample chamber is pierced to release the liquid sample.

In some embodiments, the sample collector is provided in the sample chamber and detachably combined with the sample chamber. In some embodiments, the sample collector is combined with the sample chamber. In some embodiments, the opening of the sample chamber is sealed by the sample collector and the sample chamber is allowed to retain a sample treatment liquid. In some embodiments, after the sample collector is used for collecting the sample, the sample collector is inserted into the sample chamber and the sample chamber is sealed, such that the sample collector and the sample chamber are connected into an integral structure.

In some embodiments, the sample collector and the sample chamber are independently arranged and are not detachably combined. In this case, if the treatment liquid is accommodated in the sample chamber, an opening of the sample chamber is sealed by a sealing plug. In some embodiments, the sample collector has a structure of sealing the opening, and the sample collector has a sampling area by which a powder sample and a liquid sample may be sampled. When the liquid sample is sampled, the sampling area includes an absorbent material for absorbing the liquid sample. In some embodiments, the absorbent material can be compressed to release the liquid sample. In some embodiments, after the liquid sample is collected by the sample collector, the sealing plug is removed, the sample collector is inserted, the sampling area of the collector is located in the sample chamber, and the opening of the sample chamber is sealed by the sample collector. In some embodiments, the sample collector can be completely inserted into the sample chamber, such that the opening of the sample chamber is sealed by the sealing plug.

In some embodiments, the sample chamber is a chamber with one open end and the closed other end. When the sample collector is inserted into the chamber from the opening, the opening of the tube is sealed by the sample collector or the opening of the sample chamber is sealed by the sealing plug. In some embodiments, the closed end is sealed by an easy-to-pierce sealing film. When the sample chamber is inserted into the accommodating chamber, the sealing film is pierced to release the liquid sample.

In some embodiments, the accommodating chamber is used for receiving the sample chamber, such that the sample chamber can be pierced in the accommodating chamber, and the liquid sample in the sample chamber can be released. In some embodiments, the accommodating chamber includes a piercing element or a piercing structure, and the sealing film is pierced by the piercing element. The accommodating chamber includes a tube, and the sample chamber can be inserted into the tube. In some embodiments, the tube of the accommodating chamber includes a structure with one open end and the other end having a bottom, and the bottom includes the piercing element that protrudes upward. In some embodiments, a distance between the top of the piercing element and the opening of the tube is smaller than a distance between the bottom of the sample chamber and the opening. Thus, when the sample chamber is inserted into the tube of the accommodating chamber, the piercing element can pierce the sealing film at the bottom of the sample chamber. In some embodiments, after the piercing element pierces the sealing film, a part of the piercing element enters the sample chamber and forms a pierced opening in the sealing film. In some embodiments, when the sample collector is inserted into the sample chamber, the opening is hermetically sealed, thus forming a hermetically sealed space in the sample chamber; the space has air and the treatment liquid (if any) therein. When the piercing element enters the sample chamber, a specified pressure is given to the gas, such that the increased pressure causes the liquid sample to flow out through the pierced sealing film.

In some embodiments, an outer diameter of the piercing element is slightly smaller than an inner diameter of the sample chamber, such that the liquid sample in the sample diameter can flow out along the piercing element after the piercing element enters the sample chamber in all or in part. In some embodiments, a diversion channel is provided in the piercing element, and when the piercing element is inserted into the sample chamber, the liquid sample flows out through the diversion channel. In some embodiments, when the piercing element enters the sample chamber, a part of the diversion channel also enters the sample chamber, such that the liquid sample is easy to flow out along the diversion channel.

In some embodiments, the piercing element includes a sharp structure for piercing the sealing film. In some embodiments, the accommodating chamber further includes a blade structure for laterally cutting, notching and tearing the sealing film. When the sealing film contacts the blade structure, the blade structure can laterally cut, shear, notch and tear the sealing film, such that openings, cuts, indirect openings and tears are formed within a wider range of the sealing film. In some embodiments, the sharp structure of the piercing element pierces the sealing film along the longitudinal direction of the sample chamber to form the pierced opening. After the pierced opening is formed, the blade structure laterally cuts, shears, notches and tears the sealing film from the pierced opening to form a wider range of openings, and even a piece of the sealing film or a part of the sealing film can be cut from the sealing film, and the cut sealing films do not cover the bottom of the sample chamber. In some embodiments, the blade structure is located on the piercing element. In other embodiments, the blade structure is arranged near the piercing structure. In some embodiments, the sharp structure of the piercing element and the blade structure are arranged in such a way that the sharp structure is in contact with the sealing film and then the blade structure is in contact with the sealing film. Therefore, in some embodiments, in terms of a distance between the sealing film and the sharp structure and between the sealing film and the blade structure, a distance between the sharp structure and the surface of the sealing film is smaller than that between the blade structure and the surface of the sealing film. In some embodiments, if both the blade structure and the sharp structure of the piercing element are arranged at the bottom of the accommodating chamber, the position of the sharp structure of the piercing structure is higher than that of the blade structure.

In some embodiments, when the blade structure is arranged near the piercing element, the blade structure is connected to the piercing element. In some embodiments, the blade structure is connected to the projection plane of the sharp structure of the piercing element, so when the sharp structure pierces the sealing film to form the pierced opening, the blade structure can tear, shear and cut laterally the sealing film from the pierced opening.

In some embodiments, the sample chamber enters the accommodating chamber in an insertion and/or rotation manner. When the sample chamber fits with the accommodating chamber in a relative rotation or rotational manner, the bottom of the sample chamber contacts the piercing element in the accommodating chamber, and then the piercing element pierces the sealing film. When or after the piercing element pierces the sealing film, the piercing element is used for laterally tearing, shearing or cutting the sealing film from the pierced opening, such that the pierced opening is enlarged, or the sealing film is sheared to remove the part of the sealing film from the bottom of the sealed sample chamber, more openings are exposed at the bottom of the chamber, and then the interference of the remaining sealing film on the flow of the liquid is avoided. In the embodiment, the piercing element can implement the above operation when including only a sharp piercing structure. Of course, in a preferred embodiment, the blade structure can still be arranged along a longitudinal direction of the piercing structure. In some embodiments, the accommodating chamber remains fixed, while the sample chamber rotates in the accommodating chamber or enters the accommodating chamber in a rotation or rotational manner.

In some embodiments, the device further includes a testing chamber, and the testing chamber includes a testing element, and the testing element can test the presence or absence or quantity of the analyte in the liquid sample. In some embodiments, the accommodating chamber is arranged in the testing chamber. In some embodiments, the accommodating chamber has a cover used for sealing the opening of the testing chamber. Therefore, the testing chamber is enclosed by the bottom, the opening and side walls; and a carrier for bearing the testing element is provided on the side wall. In some embodiments, the accommodating chamber is a tube, the bottom of the tube is close to the bottom of the testing chamber, and the cover seals the opening of the testing chamber. In some embodiments, the sample chamber is inserted into the accommodating chamber of the tube, and the opening of the tube is sealed by the sample chamber. In some embodiments, the sealing includes liquid sealing and gas sealing. In some preferred embodiments, the sealing is the gas sealing.

In some embodiments, the testing chamber and the accommodating chamber are fixedly combined. Thus, the accommodating chamber and the testing chamber are fixed, and the sample chamber and the sample collector are detachably combined. Of course, when the sample collector needs to collect the sample again, it is kept dry and inserted into the sample chamber after collecting the sample, such that the collection area of the sample collector is placed in the treatment liquid of the sample chamber. In this case, the sample collector and the sample chamber are separately arranged and separately packaged, and the sample collector and the sample chamber are combined only after the sample collector is used for collecting the sample. In the embodiment, the opening of the sample chamber is sealed by the sealing plug to avoid the leakage of the treatment liquid.

In some embodiments, after the sample collector is used for collecting the sample, it is inserted into the sample chamber and used for sealing the opening of the sample chamber. When the sample chamber is inserted into the accommodating chamber, the opening of the accommodating chamber is sealed by the sample chamber. In some embodiments, the sample chamber enters the accommodating chamber through thread rotation. In some embodiments, the piercing element also pierces the sealing film in the rotation manner and enters the bottom of the sample chamber. If the piercing element directly pierces the sealing film and enters the sample chamber in the rotation manner, the sealing film can be pierced to the maximum extent to form an opening, thereby helping the liquid to flow down from the bottom of the sample chamber. This is due to the fact that the pierced part of the sealing film will contact with the piercing element if the piercing element directly pierces the sealing film, thus preventing the liquid from normal downward flow. If the piercing element pierces the sealing film in the rotation manner, the piercing element has the function of piercing the sealing film and the function of cutting and shearing, so the sealing film can be cut open.

In some embodiments, a receiving tube is provided with an opening at the piercing element, and the height of the opening is higher than that of the piercing element. In some embodiments, the piercing element is located on a piston, and the diameter of the piston is equivalent to the inner diameter of the bottom of the sample chamber, while the piercing element and the blade structure are arranged at the end of the piston. When the piston enters the sample chamber in the relative rotation manner, the sealing film is cut by the rotating piercing element. In some embodiments, the sealing film covers the bottom of the sample chamber, and can be cut along the inner edge of the bottom of the sample chamber, such that the sealing film can be completely separated from the bottom by the force of the piston entering the sample chamber, and the liquid can flow out of the sample chamber freely. In some embodiments, a diversion channel is provided on the side wall of the piston. The treatment liquid in the sample chamber flows out along the diversion channel. In some embodiments, the flowing liquid flows into the bottom of the testing chamber to contact with the sample application part of the testing element.

In some embodiments, the sample collector is inserted into the sample chamber through thread rotation, and then the sample collector is fixed with the sample chamber. In some embodiments, the sample chamber is communicated with the sample collector and is inserted into the accommodating chamber in the rotation manner, and fixed together with the accommodating chamber. When the sample chamber is fixed together with the accommodating chamber, the sample chamber cannot be separated from the accommodating chamber. In some embodiments, the sample chamber and the accommodating chamber have screw threads, and are directly meshed with each other through the screw threads, such that the sample chamber enters the accommodating chamber through meshing of screw threads and relative rotation. After the screw threads are meshed with each other, they are not easy to reverse and the sample chamber is not separated from the accommodating chamber, such that the sample chamber and the accommodating chamber are connected into an integral structure.

In some embodiments, the invention provides a device for testing an analyte in a liquid sample, and the device includes a sample chamber for receiving a sample collector, where the sample chamber and the sample collector are detachably combined; a testing chamber having a testing element therein, where the testing element is used for testing an analyte in the sample chamber; and an accommodating chamber for receiving the sample chamber, where the accommodating chamber is in fluid communication with the testing chamber.

In some embodiments, an easy-to-pierce sealing film is provided at the bottom of the sample chamber, a piercing element is provided at the bottom of the accommodating chamber, and when the sample chamber is inserted into the accommodating chamber, the piercing element pierces the sealing film, thereby releasing the liquid sample in the sample chamber.

In some embodiments, the sample chamber further includes a solution for sample treatment.

In some embodiments, the sample chamber further includes the sample collector, and the collector includes a cover and a collecting head, where the cover is used for sealing the opening of the sample chamber, and the collecting head is located in the sample chamber. In some embodiments, the samples are feces, saliva and gastric juice samples. In some embodiments, the analyte is hemoglobin and helicobacter pylori.

In some embodiments, the bottom of the testing chamber is in fluid communication with the bottom of the accommodating chamber.

In some embodiments, the piercing element of the accommodating chamber is located on a piston, and when the sample chamber is pierced, the piston enters the sample chamber, forcing the liquid to flow into the bottom of the accommodating chamber through the piston. In some embodiments, the accommodating chamber has an opening at the piston, and the liquid sample flows into the bottom of the testing chamber through the opening. In some embodiments, the piston has a channel for draining the liquid sample from the sample chamber to the testing chamber to contact with the testing element, thereby completing the test of the analyte in the liquid sample.

According to a third aspect of the invention, a method for testing an analyte in a liquid sample is provided, and includes: providing a device, where the device includes a sample chamber in which a treatment liquid and a sample collector inserted into the sample chamber are provided; and a testing chamber including a testing element and a tube for accommodating the sample chamber, where the bottom of the tube is close to the testing chamber, the tube includes a cover with an opening for inserting the sample chamber, and the cover seals the opening of the testing chamber;

The sample collector is removed from the sample chamber, the sampling end of the sample collector is used for sampling and then inserted into the sample chamber.

In some embodiments, the sample chamber has a partition by which the sample chamber is divided into two chambers, the treatment liquid is accommodated in one of the two chambers and a part of a sampling rod is accommodated in the other chamber thereof. In some embodiments, a sealing element is provided on the sampling rod, and when the sampling rod passes through the partition, the partition is sealed by the sealing element, such that the chamber for accommodating the liquid is sealed and preferably hermetically sealed.

In some embodiments, the sample chamber is inserted into the accommodating embodiments in a rotation manner. In some embodiments, when the sample chamber and the accommodating chamber are fixed together, the sample chamber cannot be removed from the accommodating chamber or separated from the accommodating chamber.

In some embodiments, a piercing element is provided at the bottom of the accommodating chamber, and can pierce the sealing film of the sealing chamber. In some embodiments, the piercing element is located on the surface of the piston. The sample chamber is inserted into the accommodating chamber in the rotation manner, and the sealing film is opened by the piercing element on the surface of the piston, and the piston is allowed to enter the sample chamber. In some embodiments, when the piston enters the sample chamber, a part of the treatment liquid flows into the bottom of the testing chamber.

In some embodiments, the sample chamber is combined with the sample collector at the beginning. The testing chamber and the accommodating chamber are combined and inseparable.

In some embodiments, when testing is needed, the sample collector is removed from the sample chamber for sample collection. In some embodiments, the collecting head is allowed to adsorb liquid or solid samples, or semi-solid samples. After the collection, the sample collector is inserted into the sample chamber again, and the sampling head with the collected sample is immersed in the treatment liquid of the sample chamber.

### Beneficial effect

According to the invention, through combination of the sample chamber and the accommodating chamber, collection and detection can be separately performed, and self-testing at home can also be realized. The step-by-step collection and detection are particularly suitable for the elderly, and the general elderly can collect liquid samples on their own, but are not skillful in detection operation. However, they can allow their own family members to perform such operation or send the collected samples to the infirmary to perform such operation, thereby reducing operation errors. In addition, the samples can be collected separately, and tested by a special structure during the test, thus ensuring the objectivity of the test.

### BRIEF DESCRIPTION OF THE DRAWINGS

FIG. 1 is a schematic diagram showing a structure of a testing element according to a specific embodiment of the invention.
FIG. 2 is a schematic diagram showing a three-dimensional structure of a testing element according to a specific embodiment of the invention.
FIG. 3 is a structural exploded view of a testing device according to a specific embodiment of the invention.
FIG. 4 is a schematic diagram showing a three-dimensional structure of a testing device according to a specific embodiment of the invention (combination of an accommodating chamber and a testing chamber).
FIG. 5A is a schematic diagram showing a three-dimensional structure of combination of a collector and a sample chamber according to a specific embodiment of the invention (detachable combination of the collector and the sample chamber).
FIG. 5B is a schematic diagram showing a three-dimensional structure of combination of a collector and a sample chamber according to a specific embodiment of the invention.
FIG. 6 is a schematic diagram showing a longitudinal cross-section structure of combination of a sample chamber and a collector according to a specific embodiment of the invention.
FIG. 7 is a schematic diagram showing a three-dimensional structure of an accommodating chamber for accommodating a sample chamber according to a specific embodiment of the invention.
FIG. 8 is a schematic diagram showing a three-dimensional structure of an accommodating chamber for accommodating a sample chamber according to a specific embodiment of the invention.
FIG. 9 is a schematic diagram showing a three-dimensional structure of an accommodating chamber for accommodating a sample chamber according to a specific embodiment of the invention.
FIG. 10 is a schematic diagram showing a cross-section structure of an accommodating chamber for accommodating a sample chamber according to a specific embodiment of the invention.
FIG. 11 is a schematic diagram showing a cross-section structure of an accommodating chamber for accommodating a sample chamber according to a specific embodiment of the invention.
FIG. 12 is an enlarged schematic diagram of a partial structure of an accommodating chamber according to a specific embodiment of the invention.
FIG. 13 is a partial schematic diagram of a piston element according to a specific embodiment of the invention.
FIG. 14A is a partial schematic diagram of a piston element with a piercing element according to a specific embodiment of the invention. FIG. 14B is an enlarged schematic diagram of a piercing structure.
FIG. 15 is a partial schematic diagram of a piston element with a piercing element according to a specific embodiment of the invention.
FIG. 16A is a schematic diagram showing a structure where a sealing film seals a bottom of a sample chamber. FIG. 16B is a schematic diagram showing a structure that a piercing element fits with a blade structure to cut a sealing film. FIG. 16C is a schematic diagram showing a structure where a sealing film is cut from a bottom of a sample chamber. FIG. 16D is a schematic diagram of cutting of a sealing film.
FIG. 17A is a schematic diagram of a sample chamber with a collector being inserted into an initial position of an accommodating chamber according to a specific embodiment of the invention.
FIG. 17B is a schematic diagram showing a longitudinal cross-section structure where a sample chamber with a sample collector is inserted into an initial position of an accommodating chamber according to a specific embodiment of the invention (a sealing film of the accommodating chamber does not contact with a piercing element).
FIG. 18A is a schematic diagram showing a state where a sample chamber with a sample collector is inserted into an accommodating chamber and pierced by a piercing element according to a specific embodiment of the invention.
FIG. 18B is a schematic diagram showing a longitudinal cross-section structure where a sample chamber with a sample collector is inserted into an accommodating chamber and pierced by a piercing element according to a specific embodiment of the invention (a sealing film of the accommodating chamber contacts with the piercing element).
FIG. 19A is a schematic diagram showing a structure where a sample chamber with a sample collector is inserted into an accommodating chamber and pierced by a piercing element according to a specific embodiment of the invention (a sealing film of the accommodating chamber contacts with the piercing element and a piston is allowed to enter the accommodating chamber).
FIG. 19B is a schematic diagram showing a structure where a sample chamber with a sample collector is inserted into an accommodating chamber and pierced by a piercing element according to a specific embodiment of the invention.
FIG. 20 is a schematic diagram showing a three-dimensional structure of a testing device according to a specific embodiment of the invention.
FIG. 21 is a schematic diagram showing a three-dimensional structure of a testing device according to a specific embodiment of the invention.
FIG. 22 is a structural schematic diagram showing a cross-section structure of a testing device according to a specific embodiment of the invention.
FIG. 23 is a schematic diagram showing a structure of a testing device that has been tested (a sample chamber enters and exits an accommodating chamber).
FIG. 24 is a schematic diagram showing a cross-section structure of a testing device (a sample chamber is inserted into an accommodating chamber to start a test).

### DETAILED DESCRIPTION OF THE INVENTION

The structures involved in the invention or the technical terms used are further explained below. Unless otherwise specified, they shall be understood and explained according to the general terms commonly used in the art.

### Detection

Detection means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein, or a polymer. In addition, detection means that the amount of a substance or material is tested. Further, assay also means immunoassay, chemical assay, enzyme assay, and the like.

### Samples

Samples tested by the testing device of the invention include biological fluid samples (for example, case fluid or clinical sample). Liquid samples or liquid specimens may be derived from solid or semi-solid samples, including feces, biological tissues and food samples. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological specimen is urine; and preferably, the biological specimen is saliva. Food samples include food processing substances, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other wastewater.

An appropriate testing device according to the invention can be used for testing any analyte. Preferably, the testing device of the invention may be used for testing small drug molecules in saliva and urine, or may be used for testing hemoglobin and helicobacter pylori antigen. Of course, the samples detected by the testing device of the invention may be any samples of the above forms, regardless of being solid or liquid at the beginning, provided that these liquids or liquid samples can be absorbed by the sample application area 905 of the testing element 900. Generally, the sample application area 905 is made of an absorbent material, and liquid samples or liquid specimens can be absorbed by the capillary or other characteristics of the material of an absorption element, such that the liquid sample can flow in the sample application area. The material of the sample application area 905 may be any material capable of absorbing liquid, such as sponge, filter paper, polyester fiber, gel, non-woven fabric, cotton, polyester film, and yarn. Of course, the sample application area is not necessarily made of absorbent materials, and can be made of non-absorbent materials. However, it also has the function of transferring the liquid sample under capillary actions.

### Liquid, liquid sample and treatment liquid

The term "liquid" as used herein can have different meanings in different contexts. The liquid may include a liquid sample in the form of a liquid, or may include a treatment liquid for treating a liquid sample, for example, a solid sample is allowed to be dissolved in the treatment liquid, or the liquid sample is allowed to be dissolved in the treatment liquid, thereby forming a mixed solution formed by the treatment liquid and the liquid sample, or the treatment liquid and the solid sample. The sample treated by the treatment liquid may also be called the liquid sample and may be the liquid sample, the solid sample or a powder sample. When the liquid is used as the treatment liquid, generally the liquid here is water as a solvent, and the treatment liquid may include other reagents to improve the test performance, for example, a PH regulation reagent, some reagents to remove impurities from the samples, or a reagent to dissolve the samples, but does not include a target analyte. Therefore, when a solution is accommodated in the sample chamber 400 (in absence of the sample), it is generally the treatment liquid and can dissolve, elute and improve the reaction performance. The solution can dissolve the sample collected by the sample collector 200. Of course, the sample can be the liquid sample, the semi-solid sample, the solid sample or the semi-solid sample between solid and liquid samples (for example, a gelatinous sample including a liquid). When the sample collector 200 is inserted into the sample chamber 400, the sample on the sample collector contacts with the treatment liquid in the sample chamber, such that the sample can be dissolved in the treatment liquid, can be deemed to be the liquid sample or the sample treated by the treatment liquid; especially, if the sample contains the target analyte, the target analyte can be dissolved in the treatment liquid. Of course, if the liquid itself is in the form of the liquid sample, the treatment liquid may or may not be accommodated in the sample chamber in advance. When the liquid sample is urine, the urine directly flows into the sample chamber. When the liquid sample is in the form of saliva, nasal mucus, sputum and other liquids, the treatment liquid can be stored in the sample chamber in advance to treat these liquid samples. For example, if the sample collector is used for collecting a fecal sample, the fecal sample is dissolved in the treatment liquid in the sample chamber 400. For example, when the sample collector is used for collecting the powder sample, the treatment liquid is accommodated in the sample chamber 400, such that the powder sample is dissolved in the treatment liquid to form a liquid sample. Therefore, if the sample needs to contain the treatment liquid, the treatment liquid can be stored in the sample chamber in advance, and the treatment liquid is mixed with the sample to form a mixed liquid that can be called a sample solution. Of course, as mentioned above, if the treatment liquid is not accommodated in the sample chamber, the liquid sample is directly sent into the sample chamber for storage and can also be deemed to be the liquid sample.

### Downstream and upstream

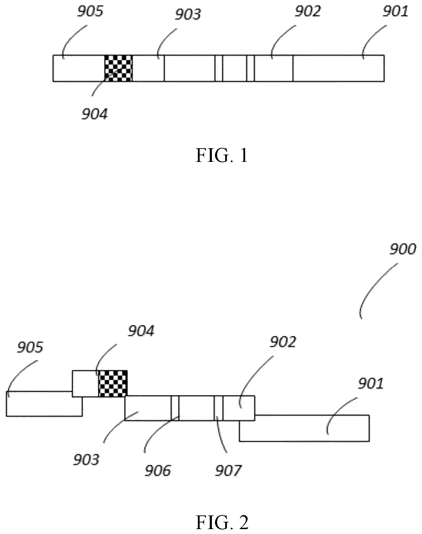

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to facilitate the flow of a liquid, a liquid may overcome gravity to flow towards an opposite direction to the gravity; and in this case, downstream or upstream is divided according to a flow direction of the liquid. For example, in the testing device of the invention, after a diversion element receives the liquid sample, fluid can flow from the diversion element to a sample application area or a sample application pad of two testing elements, and then liquid flowing to the sample application pad flows to a downstream label pad and is mixed with the marked label; and the mixture flows to a downstream testing pad through a transition pad, where a testing area on the testing pad is located upstream of a testing result control area, such that the mixture finally flows to an absorption pad on a downstream absorption area. The testing area may be a polyester fiber film, and the diversion element may be a glass fiber, a polyester chip, and a polyester film. In this case, the diversion element is located at the upstream of the label area of the testing element. The specific structure of the testing element is a structure 90 as shown in FIG. 1 and FIG. -2. Liquid on a part of the sample application pad flows mainly by a capillary force.

### Gas communication or liquid communication

Gas communication or liquid communication means that liquid or gas can flow from one place to another. In the flow process, the liquid or gas may pass through some physical structures that play a guiding role. The "passing through some physical structures" here means that liquid passes through the surface of these physical structures or their inner space and flows to another place passively or actively, where passivity is usually caused by outer forces, such as flow under the capillary action and the action of air pressure. The flow here may also be a flow due to self-action (gravity or pressure) of the liquid or gas, and may also be a passive flow. The fluid under the action of air pressure may be a forward flow, or also a reverse flow; or a fluid is caused to flow to another position from a position under the action of air pressure. The communication here does not mean that a liquid or a gas is necessarily present, but indicates a relationship or state between two objects under some circumstances. If a liquid is present, it can flow from one object to another. Here it means the state in which two objects are connected. In contrast, if there is no liquid or gas communication state between two objects, and a liquid exists in or on one object but is unable to flow into or on another object, it is a non-communication, non-liquid communication or non-gas communication state.

### Testing element

The "testing element" used here refers to an element that can be used for testing whether a fluid sample or a fluid specimen (a liquid sample or a liquid specimen) contains an interested analyte. Such testing can be based on any technical principles, such as immunology, chemistry, electricity, optics, molecular science, nucleic acids, and physics. The testing element can be a lateral flow test strip that can detect a variety of analytes. Of course, other suitable testing elements can also be used in the invention. In the invention, the testing element and the "lateral flow testing element, or test strip" can be used interchangeably, indicating same meanings.

Various testing elements can be combined for use in the invention. One form of the testing elements is a test strip. The test strips used for analyzing the analyte (such as drugs or metabolites that show physical conditions) in samples can be of various forms such as immunoassay or chemical analysis. A non-competitive or competitive analysis mode may be used for the test strips. A test strip generally contains a water absorbent material that has a sample application area, a reagent area, and a testing area. Fluid or liquid samples are added to the sample application area and flow to the reagent area under the capillary action. If analyte exists in the reagent area, samples will bind to the reagent. Then, the samples continue to flow to the testing area. Other reagents such as molecules that specifically bind to the analyte are immobilized in the testing area. These reagents react with the analyte (if any) in the sample and bind to the analyte in this area, or bind to a reagent in the reagent area. The label used for displaying the detection signal exists in the reagent area or the detached label area.

In a typical non-competitive analysis mode, if a sample contains the analyte, a signal will be generated; and if not, no signal will be generated. In a competitive method, if no analyte exists in the sample, a signal will be generated; and if the analyte exists, no signal will be generated.

The testing element may be a test strip, which may be made of a water absorbent material or non-water absorbent material. The test strip may contain several materials used for delivery of liquid samples. One material of the test strip can cover the other material thereof. For example, the filter paper covers the nitrocellulose membrane. One or more materials may be used in one area of the test strip, and one or more other different materials may be used in the other area. The test strip can be stuck to a certain support or on a hard surface for improving the strength of holding the test strip.

The analyte is detected through a signal generating system. For example, one or more enzymes that specifically react with this analyte is or are used, and the above method of fixing a specific binding substance on the test strip is used for fixing the combination of one or more signal generating systems in the analyte testing area of the test strip. The substance that generates a signal may be in the sample application area, the reagent area or the testing area, or on the whole test strip, and one or more materials of the test strip may be filled with this substance. The solution containing a signifier is added onto the surface of the test strip, or one or more materials of the test strip is or are immersed in a signifier-containing solution. The test strip containing the signifier solution is dried.

Various areas of the test paper or the lateral flow test strip 900 of the invention can be disposed as follows: sample application area 905, label area 904, and testing area 902, where the testing area includes a testing result area 906 and a testing result control area 907. The control area 907 is located behind or downstream of the testing area 906. All areas can be arranged on one test strip that is only made of one material. Alternatively, different areas may be made of different materials. Each area can be in direct contact with the liquid sample, or different areas are arranged according to the flow direction of liquid sample; and a tail end of each area is connected and overlapped with the front end of another area. Materials used can be those with good water absorption such as filter paper, glass fibers or nitrocellulose membranes. The test strip may also be in other forms.

The nitrocellulose membrane test strip is commonly used, that is, the testing area includes a nitrocellulose membrane (NC) on which a specific binding molecule is immobilized to display the testing result; and other test strips such as cellulose acetate membrane or nylon membrane test strips can also be used. For example, test strips and similar devices with test strips disclosed in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device with test strips disclosed in the above patents may be applied to the testing element or testing device of the invention for the test of an analyte, for example, the test of an analyte in a sample.

Test strips used in the invention may be commonly referred as lateral flow test strips. The specific structure and detection principle of the test strips are well known to a person skilled in the art in the prior art. A common test strip 900 (as shown in FIG. 1 - FIG. 2) includes a sample collection area or a sample application area 905, a label area 904, and a testing area 902; the sample collection area includes a sample receiving pad or a sample application pad; and the label area includes a label pad. The test strip may further include a water absorption area 901 to absorb the liquid sample from the nitrocellulose membrane and the water absorption area may include a water absorption pad. In some embodiments, the label area includes color particles conjugated with antibodies, and the color particles may be latex particles, gold particles, or dyes. The testing area 902 includes necessary chemical substances, such as immunoreagents or enzyme chemical reagents, all which can detect presence or absence of an analyte. The nitrocellulose membrane test strip is commonly used, that is, the testing area 902 includes a nitrocellulose membrane, and an area 906 (T-line) on which a specific binding molecule is immobilized to display the testing result; and other test strips such as cellulose acetate membrane or nylon membrane test strips can also be used. Of course, in the downstream of the testing area, there may also be a testing result control area 907 (C-line); generally, test strips appear on the testing result control area and the testing area in the form of a horizontal line, namely, a test line or a control line. Such test strips are conventional. Of course, they can also be other types of test strips for detection under the capillary action. In addition, there are dry chemical reagent components on common test strips, for example, an immobilized antibody or other reagents. When the test strip contacts a liquid, the liquid flows along the test strip under the capillary action, and the dry reagent components are dissolved in the liquid and treated in a next area, and the dry reagents react in the area for necessary detection. The liquid flow mainly relies on the capillary action. Here, all of the test strips can be applied to the testing device of the invention or can be disposed in contact with the liquid samples in a testing chamber or used for detecting the presence or absence of an analyte in the liquid samples that enter a testing chamber, or the quantity thereof.

In addition to the foregoing test strip or lateral flow test strip which is used for contacting with the liquid sample to test whether the liquid samples contain analytes. The testing element of the invention may be used as a testing device by itself to detect an analyte in a sample. Therefore, the testing device here is equal to a testing element. For example, after mixed with a treatment liquid, the liquid sample is detected with a testing element directly, specifically described as follows: When a receiving device is described to treat a liquid sample, the testing element may be used for detection alone.

### Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzodiazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The testing device of the invention may also be used for detecting drugs belonging to a medical use but easy to be taken excessively, such as tricyclic antidepressants (imipramine or analogues) and acetaminophen. These drugs are metabolized into micromolecular substances after being absorbed by human body. These micromolecular substances exist in blood, urine, saliva, sweat and other body fluids or in some body fluids.

For example, the analyte detected by the invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus*, *Salmonella*, *Fusiformis*, *Camyplobacter genus*, *L. monocytogenes*, *Vibrio*, or *Bacillus cereus*) and substances related with physiological features in a urine sample, such as pH and specific gravity. Chemical analysis of any other clinical urine may be performed by lateral flow test in combination with the device of the invention. Such chemical analysis can be also used for testing the presence of virus antigens, such as COVID-19 antigen and influenza antigen.

### Detachable combination

A detachable combination means that two components are connected in several different states or positional relationships. For example, with two components being physical components, they can be separated at the beginning and then connected or combined in an appropriate first case, and separated in an appropriate second case. Physically, such separation is spatial separation without contact. Alternatively, the two components are combined at the beginning, and can be physically separated from each other when appropriate. Alternatively, two objects are separated at the beginning, combined to achieve a specified function if necessary, then separated, or later combined again for a purpose. In short, combination or separation of two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component; and a detachable combination or separation is made between the first component and the third component. In addition, for the combination, two objects themselves can be detached or can be indirectly combined by other objects.

### Carrier including testing element

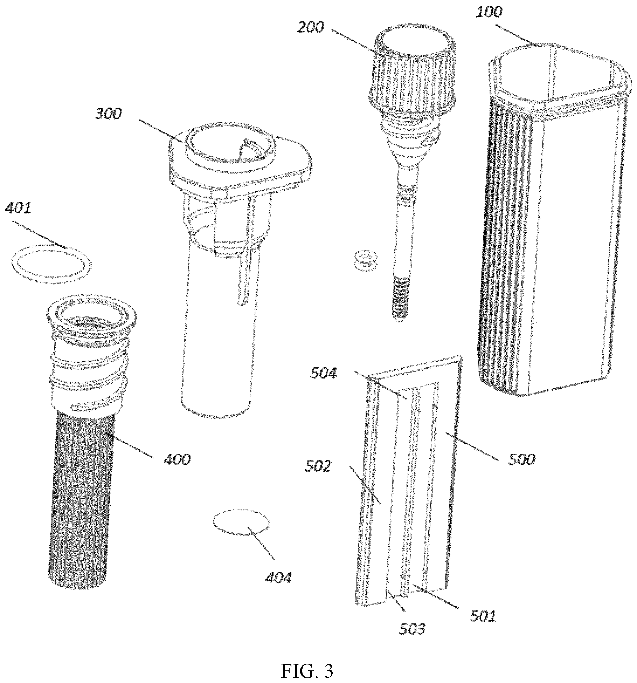

In some specific embodiments, the testing element may be also disposed on some carrier elements 500; and the carrier elements include the testing element to complete the detection and assay of the analytes in liquid samples. Therefore, in some embodiments, the testing device includes a carrier 500, and the carrier is provided with a testing element 900. In some embodiments, the carrier of the invention is a housing used for bearing or accommodating the testing element; the carrier element does not participate in the detection directly by itself, but serves as a carrier or housing used for bearing or accommodating the testing element. For example, as shown in FIG. 3, a carrier 500 is provided, where grooves 501 are provided in the carrier and are configured to accommodate the testing element 900. One of the carriers can be provided with a plurality of grooves, and each of the grooves is provided with one testing element. Generally, the sample application area of the testing element is located at or extends from one end of the groove having an opening 503. After the testing element 900 is provided in the groove, a transparent film (not shown)covers the surface of the carrier 500, and then the carrier 500 is inserted into a testing chamber 100. The testing chamber has two faces opposite a plane. The carrier 500 is inserted into the testing chamber 100 and rests on the surface of the plane 102, while the face having the testing element rests on the plane, such that testing results on the testing element can be read through a transparent surface of the testing chamber during the test. Of course, the transparent plane of the testing chamber 100 can also be directly placed on a scanner to be scanned, and the testing results are recorded. In some embodiments, each of the grooves in the carrier 500 has a protruding structure at one end thereof, and the protruding structure allows the testing element to be fixed in the groove. Generally, the convex structure is the position of the water absorption pad stuck on the water absorption area of the testing element.

### Testing chamber including testing element

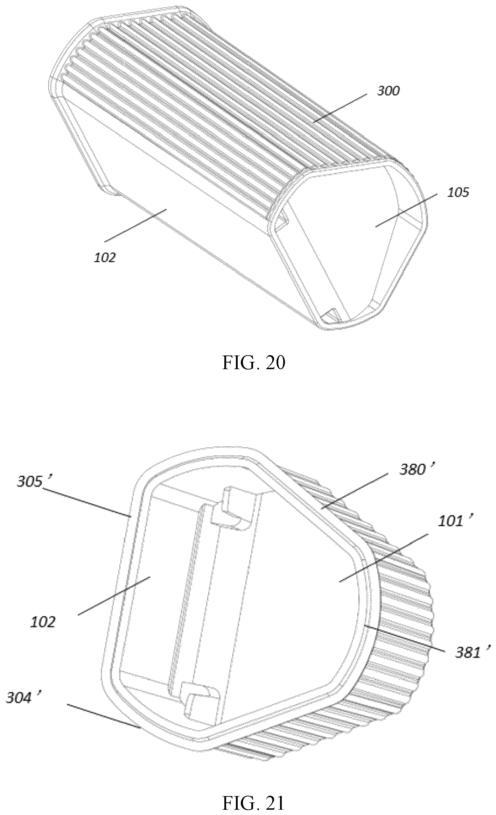

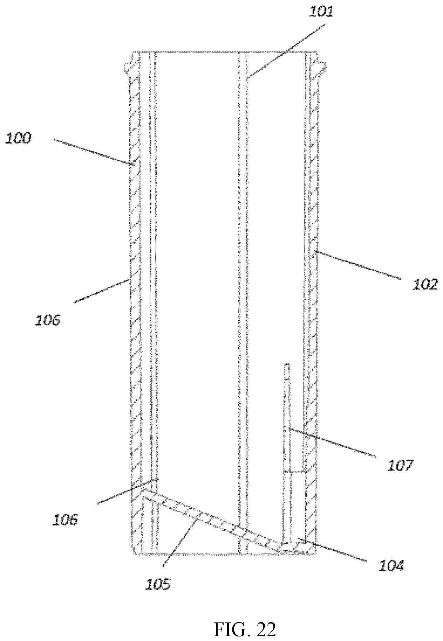

In some specific embodiments, as shown in FIG. 3, and FIG. 20 - FIG. 22, the invention provides a testing chamber 100, and the testing chamber 100 includes a testing element 900, and the testing element may be provided in one or in plurality. In some embodiments, the testing element 900 is provided on a carrier 500, and the carrier 500 includes a plurality of grooves, each of the grooves is provided with a test strip, and the carrier is located in the testing chamber. As shown in FIG. 1, FIG. 22-FIG.24, the testing chamber 100 is enclosed by an opening 101, side walls and a bottom 105. The side walls are enclosed into an irregular shape, for example, a generally triangular shape. A transparent plane 102 is provided on the side walls, a fixing structure 107 is provided inside the testing chamber and symmetrically arranged on the back of the plane 102 and extends upward from the bottom of the testing chamber. The carrier 500 with the testing element 900 is arranged between the fixing structure and the plane 102, and the groove in the carrier faces the plane 102, such that the testing area and the testing control area of the testing element 900 can be seen through the plane 102. Meanwhile, the outlet 503 of the groove of the carrier is close to the bottom 105 of the testing chamber. Preferably, at the groove 104, for example, the end of the sample application area 905 of the testing element 900 is in contact with the bottom of the groove 104, such that the liquid sample or the liquid including the sample is collected at the groove 104 and contacts with the sample application area of the testing element 900, and the assay of the analyte is completed. In some embodiments, the bottom 105 is an inclined plane; the position of a part of the inclined plane 106 of the plane 108 opposite to the connecting plane 102 is higher than the position of the groove 104, and the inclined plane 106 connects to the plane 108 and the groove, such that when the liquid sample flows out from the bottom of the accommodating chamber 300, it flows to a relatively high position, and then flows to the groove 104 along the inclined plane for being gathered. As for why the bottom of the accommodating chamber 300 is not allowed to be close to the groove 104, the main reason is that the liquid flows out through the bottom. If the liquid directly flows from the position close to the groove 104 to the groove 104, some testing elements necessarily contact the liquid, and some testing elements contact the liquid later, such that the testing results do not appear at the same time, leading to the wrong results. On the one hand, the liquid has a diffusion process, and on the other hand, a time limit for reading the testing results of the testing elements is obtained. It is hoped that all the testing elements 900, for example, in case of multiple testing elements, will contact the liquid at the same time, such that all the testing elements can complete the test within a same time range.

### Testing chamber and accommodating chamber

In some embodiments, the accommodating chamber does not indicate a chamber, and it can be understood as a structure for receiving a sample chamber; and the structure fits with the sample chamber to release or transfer the liquid sample in the sample chamber. Therefore, the "accommodating chamber" defined herein can also be included in an embodiment of a structure that is not a chamber. The "accommodating chamber" here can be understood that: on the one hand, it is a structure for receiving the sample chamber, and the structure can release the liquid sample from the sample chamber when receiving the sample chamber, and the liquid sample will flow into the testing chamber to complete the test of the analyte in the liquid sample. On the other hand, it can be understood that the accommodating chamber and the testing chamber can be fixed together and inseparable, and of course, the accommodating chamber can be detachably combined with the testing chamber. That is to say, the accommodating chamber 300 is independently arranged at the beginning, and when the liquid needs to be released from the sample chamber, the accommodating chamber is placed in the testing chamber to release the liquid into the testing chamber. Therefore, in some embodiments, the testing chamber may further the accommodating chamber that is used for receiving the sample chamber. Of course, the accommodating chamber may be independently arranged instead of being necessarily combined with the testing chamber, so the liquid can be released from the sample chamber through the accommodating chamber, then flows into a container, and the testing element is inserted into the container and used for testing the analyte. In the specific embodiment, the accommodating chamber is not combined with the testing chamber, and is only used for transferring the liquid sample.

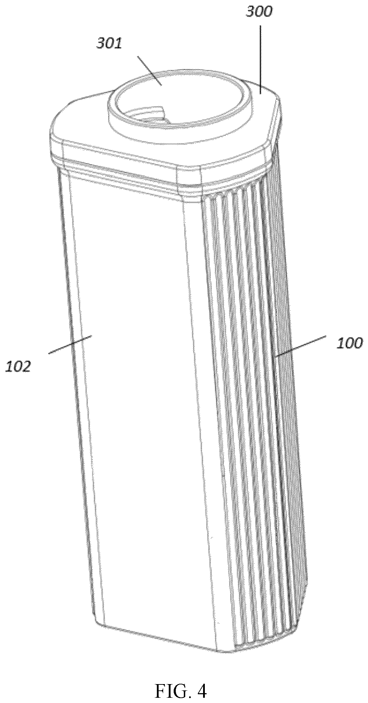

In some embodiments, the testing chamber 100 and accommodating chamber 300 of the invention are fixedly assembled together at the beginning. As shown in FIG. 4, the accommodating chamber 300 has a tube structure with an opening 301, the sample chamber 400 is allowed to be inserted into the accommodating chamber, and the tube structure is used for accommodating the sample chamber 400. The accommodating chamber of the tube is enclosed by a bottom 306 and side walls 308; and when the accommodating chamber and the testing chamber need to be assembled together, the accommodating chamber and the testing chamber are fixed together through one fixing structure. In some embodiments, the testing chamber has an opening 101, the accommodating chamber has a cover structure 303, and the cover structure fits with the opening of the testing chamber, such that the opening 101 of the testing chamber 100 is sealed and only the opening 301 of the accommodating chamber is in fluid communication with the inside of the testing chamber. Specifically, the cover of the accommodating chamber has an outer cover edge 310 and an inner cover edge 311, a cover groove 312 is provided between the inner cover edge 311 and the outer cover edge 310, and the width of the cover groove 312 is equivalent to the width of the edge of the opening 101 of the testing chamber. Therefore, the edge of the opening 101 of the testing chamber is arranged in the cover groove 312 between the outer cover edge 310 and the inner cover edge 311, the inner cover edge 311 is located in the testing chamber, and the outer cover edge 310 is located outside the testing chamber, so as to wrap the opening 101 of the testing chamber. The accommodating chamber 300 is allowed to be ultrasonically welded together with the testing chamber 100, such that the tube structure 308 of the accommodating chamber is fixedly located in the testing chamber 100. In some embodiments, the cover 303 of the accommodating chamber is similar to an irregular trapezoid and is enclosed by four edges 380, 304, 305, 381, a long edge 305 and a short edge 381 are oppositely arranged, a lateral edge 304 and a lateral edge 380 are oppositely arranged, the opening 101 of the testing chamber 100 is corresponding to the cover 303 in terms of shape and an irregular trapezoid, and the opening has a same arrangement rule as the edge of the cover. Therefore, the edge 381' of the opening 101 corresponding to the short edge 381 is located on the side wall 108, the edge 305' of the opening 101 corresponding to the long edge 305 is located on the side wall 102, the accommodating chamber 308 is close to the side wall 108, and other two lateral edges 304, 380 are corresponding to the edges 380', 304' of the opening 101 respectively. Thus, the bottom 306 of the accommodating chamber is substantially located right above one end of the high inclined plane 106 at the bottom 105 of the testing chamber 100. Thus, the liquid flowing out of the accommodating chamber directly flows onto the inclined plane 106 of the bottom 105, and then flows into the groove 104 at the bottom along the inclined plane 106; the liquid diffuses in a flowing process, and the testing element can be allowed to contact the liquid sample or the solution containing the liquid sample to complete the test of the analyte.

### Sample collector and sample chamber

The sample collector of the invention is used for collecting samples; and such samples may be liquid samples, solid samples, semi-solid samples, solid-liquid mixture samples, or powder samples. Generally, the sample collector includes a sample collection part that is referred to as the sample collection area or sample collection part for short, that is, the sample collection part directly contacts with the sample to keep it in the sample collection area or sample collection part.

In some embodiments, when the semi-solid samples including fecal samples are collected, some screw threads, holes or rough surfaces can be arranged on the sample collection part, so that the solids in the semi-solid samples are adsorbed by the screw threads and holes, and the liquid is also adsorbed by these structures.

When the sample is solid or powder, the sample collection part can be in the form of a brush, or composed of flocked cotton swabs, sponge cotton swabs or other fiber materials. Such a sample collector can be used for collecting powder samples, for example, powder of abused drugs, which can be the powder of a single drug or the mixture of multiple different drug powders. The surface can be wiped with such a brush to collect powder samples.

In some embodiments, such a sample collector can be used for collecting liquid samples. When the sample collector is used for collecting the liquid samples, the sample collection area or sample collection part is made of absorbent materials, including filter paper, fiber, sponge, polyester sponge and other absorbent materials. These materials are dry at the beginning, but become wet after absorbing the liquid samples. Such liquid samples can be secretions of saliva, sputum, nose and pharynx. The collected liquid sample is determined by the saturated absorption capacity of the absorbent material in the sample collection area of the sample collector, and may be 5 ml or 1-10 µL depending on the absorption volume or saturated absorption capacity of the absorbent material.

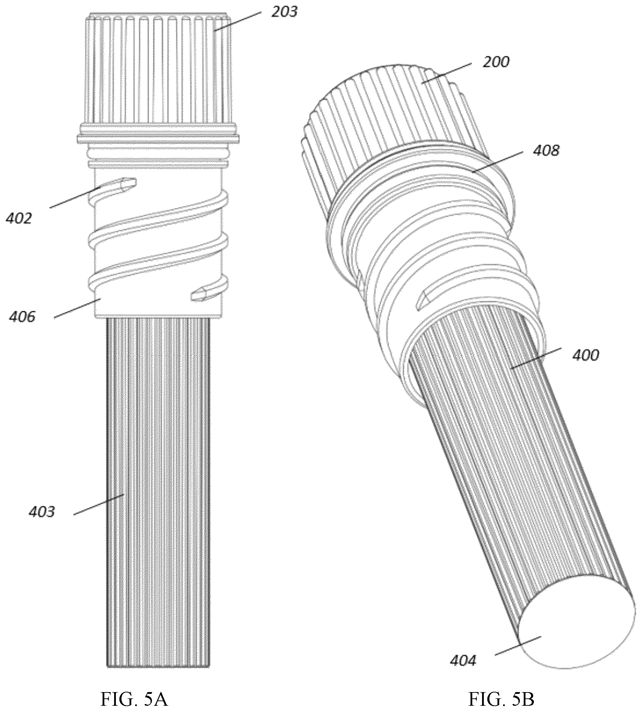

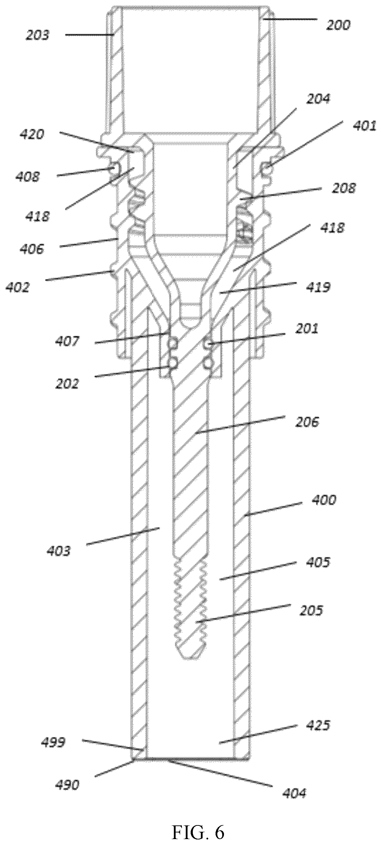

In a specific embodiment of the invention, a sample collector is provided. For example, as shown in FIG. 1 and FIG. 6, the sample collector 200 includes a sample collection area 205 with a threaded structure, and the solid or semi-solid sample is absorbed through the gap between the screw threads. The absorption area is connected by a rod 206, the rod 206 can also be connected to a threaded rod head 204, and the threaded rod head has an outer screw thread 208 that is meshed with the inner screw thread 402 of the sample chamber 400. In some embodiments, the sample collector further has a cap structure 203 that is mainly a place held by hand, such that the sample collection area 205 can contact the sample conveniently. In some embodiments, a sealing portion is provided between a collecting rod 206 and a rod head 204, and the sealing portion 207 and the structure in the sample chamber are sealed. In some embodiments, the sealing portion 207 is provided with sealing rings 201, 202, such as latex, rubber or silicone sealing rings. The sealing portion 207 fits with the specified structure of the sample chamber, and the sample chamber can be divided into two parts, one part thereof is the sealed space for storing the treatment liquid, and the other part thereof is also sealed and can be called a sample retention portion. For example, as shown in FIG. 6, when the sample collector is inserted into the chamber 400, the sample chamber 400 is divided into a lower chamber 405 and an upper chamber 418 through the sealing portion 207. A partition is arranged in the chamber of the sample chamber 400 and is similar to a short channel 406. The diameter of the channel is equivalent to the diameter of the rod 206. When the excess sample (solid sample or solid-liquid mixture sample) is obtained from the sample collection area 205, the excess sample (solid sample or solid-liquid mixture sample) will be left in the chamber 418 when passing through the channel 406, and the sample attached to the screw thread of the sample collection area 205 will enter the lower chamber 405. This prevents too many samples from entering the lower chamber 405, so as to achieve the purpose of sample quantification. In addition, the sealing portion 207 fits with a segmentation structure, such that the lower chamber 405 is sealed. The sealed chamber 405 is isolated from the outside, to prevent the missing sample or liquid in the upper chamber 418 from flowing into the lower chamber 405, and to prevent the sample in the lower chamber 405 from leaking to the outside for pollution. Therefore, generally, a space between the rod head 204 and the upper chamber 418 of the sample chamber 400 is obtained to store the excess sample. In addition, the outer screw threads 208 on the rod head 204 fit with the inner screw threads 402 of the sample outer screw threads to be fixed. The fitting of the screw threads causes the sample collector to be fixed in the sample chamber and has the sealing function, to prevent the sample in the upper chamber 418 from leaking to the outside and polluting the environment. In some embodiments, the lower chamber 405 of the sample chamber 400 is generally filled with the treatment liquid in advance, and the treatment liquid is used for dissolving the sample on the sampling area 205 and used for improving the testing performance, such as dissolving impurities and removing a protein interfering substance. When the treatment liquid is accommodated in the lower chamber 405, the sampling area 205 needs to be immersed in the treatment liquid to dissolve the sample. Such an operation is mainly to dissolve the analyte in the sample into the treatment liquid to form a liquid sample including the analyte or a diluted liquid sample. In the embodiment of the invention, the sample collector and the sample chamber are detachably combined. For example, when the sample collector is not used for collecting the sample, it is inserted into the sample chamber (as shown in FIG. 5A - FIG. 5B and FIG. 6), and the sampling area 205 is immersed in the treatment liquid in the lower chamber 405. When sampling is needed, the cap structure 203 is rotated by hand, and the sample collector is allowed to depart from the sample chamber and is used for collecting the sample such as the fecal sample by the sample collection area 205, and then is again inserted into the sample chamber; and the sample collection area 205 is immersed into the treatment liquid, to complete the sample collection.

In some embodiments, if the sample collection area 205 is the brush structure instead of the threaded structure, it can be used for collecting powdery substances, such as powdery substances of abused drugs. When the surface suspected of abused drug powder is dipped, swept and rubbed by using the brush structure or cotton swab or sponge, and then the powder can be allowed to be adsorbed on the fiber surface. Such adsorption can be electrostatic or rely on the action of surface energy, these powders are allowed to be adsorbed on the surface, the sample collection area 205 is inserted into the sample chamber and is allowed to enter the lower chamber, and the sample with the powder is retained in the lower chamber. In the embodiment, the sample collector 200 and the sample chamber 400 are independently arranged, and the sample collector 200 is not inserted into the sample chamber 400. Similarly, the treatment liquid is accommodated in the lower chamber and is used for eluting and dissolving these powder samples, thus forming a liquid sample. In the invention, the brush-like sampling area or the sampling area made of the absorbent material such as cotton swabs and flocked cotton swabs is used during the sampling. The sample collector can be packaged separately, and does not need to be inserted into the sample chamber at the beginning, and the opening 420 of the sample chamber 400 is sealed, that is, the sample chamber is sealed by not the rod head 204 of the sample collector but by other technical ways, for example a plug (not shown), such that the treatment liquid is accommodated in the whole sample chamber, and the sample chamber is independently arranged and sealed. Of course, the plug can also be made of any material, such as rubber, silica gel, and cork, and can also be sealed by the sealing film in a thermoplastic manner. Any manner that can be used for sealing the opening 420 of the sample chamber 400 can be adopted. When the sample collector is used for collecting dry powder and solid samples, the plug is removed after collection, and then the sample collector is inserted into the sample chamber. In this case, the sampling area 205 with the absorbent material (the absorbent material and the sample with dry powder) enters the sample chamber and is immersed in the treatment liquid in the lower chamber 405, such that the powder is dissolved in the treatment liquid to form a liquid sample including the powder sample. In this case, the sealing portion 207 is sealed with the partition 419 of the sample chamber, such that the lower chamber 405 where the treatment liquid is accommodated forms a sealed chamber. Of course, it can be understood that in this case, the sample chamber may not include the partition 419, for example, a structure of a pipe 406, but the sampling area with an adsorbent dry powder may be allowed to directly enter the sample chamber 400, and the opening 420 of the sample chamber may be sealed by the rod head 204 of the sample collector; alternatively, the opening of the sample chamber is sealed in such a way, for example, by the screw threads or the plug. In some embodiments, when the sample collector with the absorbent material such as cotton swabs or flocked cotton swabs is used, once sampling is completed, the whole sample collector is directly inserted into the sample chamber 400, such that the sampling area (the material that absorbs dry powder) is immersed in the treatment liquid in the lower chamber 405, the opening 420 of the sample chamber 400 is sealed with the plug (not shown), or the sampling area 205 is separated from the sample collector in a fracturing manner, and then the sampling area is kept in the treatment liquid of the sample chamber 400 and the opening 420 of the sample chamber is sealed by the sealing plug. In this case, the sample including the solution in the sample chamber forms the liquid sample and can be tested in a next step or directly sent to a testing organization for testing and assaying.

In some embodiments, the sample chamber as shown in FIG. 6 is still provided and has the upper chamber 408 and the lower chamber 405. The partition 419 is arranged between the two chambers; and a pipe 406, a channel or an opening is arranged on the partition. The sampling area of the sample collector is not of the threaded structure as shown in FIG. 6, but is made of the absorbent material, and it can absorb the liquid sample. The sample collector made of these materials such as cotton, polyester, sponge, filter paper and polyester foam can absorb the liquid sample, for example, it can go deep into the mouth to absorb a saliva sample, for example, 50-100 µl; and the absorbent material is compressible or not. The sample collector is packaged separately before used for absorbing the liquid, and includes the sample collection area for absorbing the liquid sample, the collecting rod or the rod 206, the rod head 204 and the cap structure 203 connected to the rod head. After the liquid sample is absorbed by the absorbent material at the front end, the absorbent material is inserted into the sample chamber 400 through the opening 420 of the sample chamber. In this case, the collecting head also passes through the partition and the size of the partition is smaller than that of the absorption liquid, or the absorption head absorbing the liquid is squeezed or compressed at the partition (when the absorbent material is compressible), such that the absorption head or absorption area absorbing the liquid can be compressed in time to release the liquid sample into the lower chamber 405, and the released liquid sample is mixed with the treatment liquid in the lower chamber 405. Then, the absorption head is immersed into the treatment liquid, and dissolved and eluted by the treatment liquid, such that the analyte in the liquid sample is dissolved or eluted in the treatment liquid. Of course, if the absorption area for absorbing the liquid cannot be compressed, the absorption head or the absorption area can be directly detached from the sample collector and immersed into the treatment liquid in the lower chamber 405, for example, a swab head, a sponge head, a polyester head and other materials for absorbing the liquid sample.

In some embodiments, the treatment liquid stored in the lower chamber in advance may be 5 ml to 50 µl. When the volume of the sample chamber is smaller, the volume of the treatment liquid stored is smaller. If the volume of the lower chamber of the sample chamber is larger, a great amount of the treatment liquid can be stored. When a small amount of the sample is collected by the sample collector, the treatment liquid is correspondingly reduced. For example, a tiny amount of the sample is collected by the sample collector, such as 50-100 µl, or 50-10 mg or 50-10 mcg. In this case, the treatment liquid in the sample chamber is 10-50 µl and used for dissolving the tiny amount of the sample, and the tiny amount of the sample can be tested and assayed. The tiny amount of the sample is especially suitable for a case where a small sample size is needed; for example, the saliva samples, in the test of drugs or drug abuse, a test subject has a very small amount of salivary secretions that are sampled by 5-10 µl and 50 µl for testing and assaying, but the salivary secretions are lost from the collection of the sample collector to the test on the test strip, and may not meet the requirements of the test strip for the sample; especially when the abuse of multiple drugs is tested , the requirement for the sample size is made, for example, 10 µl of the liquid is needed for each test strip; in case of 10 test strips, 100 µl of the liquid is needed, but the actual sample collected is only 10 µl; in this case, the treatment liquid needs to be provided to make the whole sample size reach more than 100 µl to meet various tests, and the treatment liquid is necessary. Although the treatment liquid may dilute the sample or the analyte, the chemical reagent of the test strip can be improved to increase detection sensitivity, for example, a method for increasing detection sensitivity described in European Patent EP2823309B1can be implemented as a part of the invention. Thus, for the sample collector, only a small amount of the absorbent material is needed; for example, for saliva test, an enough amount of the saliva sample can be collected after the sample collector slightly sweeps a mouth cavity.

All of the above are specific embodiments in which the treatment liquid is stored in the sample chamber in advance. However, in some embodiments, the treatment liquid is not stored in the sample chamber in advance, but is used for directly receiving the liquid samples, for example, urine samples; similarly, the liquid samples are received by using urine cups; then the opening 420 of the sample chamber 400 is sealed by the cover, and finally the liquid samples are sent to the laboratory for testing and assaying.

Therefore, it can be understood that in some cases, the sample chamber 400 can be detachably combined with the sample collector, or the sample collector 200 and the sample chamber 400 can be independently arranged. After the sample collector 200 is used for collecting the sample, the sample collector 200 and the sample chamber 400 can be combined together, or the sample collector 200 can be completely inserted into the sample chamber, and at least the area where the sample is absorbed or collected can directly enter the lower chamber and be immersed in the liquid. In some cases, the sample collector can be omitted, and only the sample chamber can be used for receiving the liquid samples, for example, when the liquid samples are the urine samples, 50-100 ml of the urine samples can be absorbed. In such a way, it is enough to provide the sealing element to seal the opening 420 of the sample chamber.

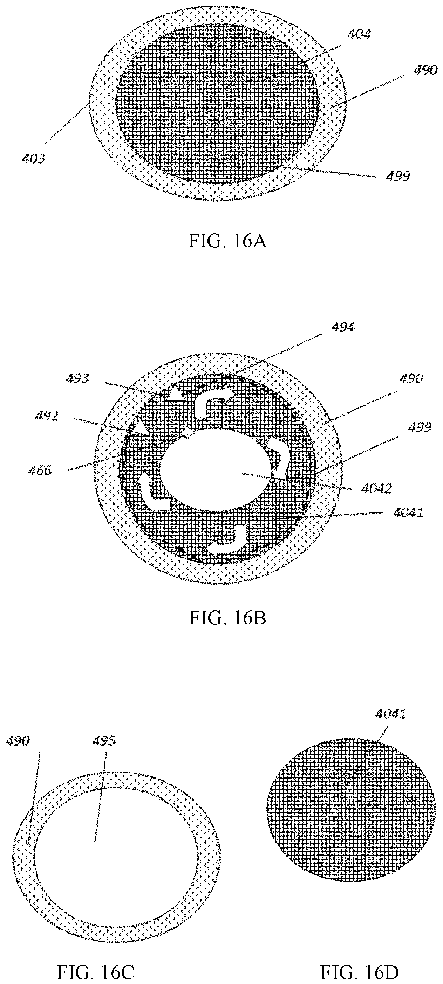

In some embodiments, the bottom 425 of the sample chamber is sealed by a sealing film 404 capable of being pierced, such piercing is to release the liquid or liquid sample in the sample chamber. The sample here may be that the liquid sample itself is not mixed with the treatment liquid, and the liquid sample here may be the liquid sample after the liquid sample collected by the sample collector is mixed with the treatment liquid in the sample chamber. The sealing film here may be a plastic sheet, an aluminum foil sheet, a plastic film, and the like. The sealing mode can be that the bottom is provided with an opening, and then a metal film covers the bottom for sealing, or one plastic sheet, such as a 1 mm thin sheet, can be injected into the bottom during plastic injection; thus, when the treatment liquid is added from the opening 420 of the sample chamber, the treatment liquid is stored in the lower chamber 405 without being leaked. When necessary, the film at the bottom of the sample chamber is pierced by the piercing element. For example, as shown in FIG. 16A, the sample chamber has a bottom covered by a layer of the sealing film 404, and the sealing film is bonded to the edge 490 of the side wall 402 of the sample chamber to seal the bottom of the sample chamber. The film and the edge 490 at the bottom of the sample chamber can be bonded together by glue, ultrasonic heating and welding.

### Accommodating chamber and sample chamber

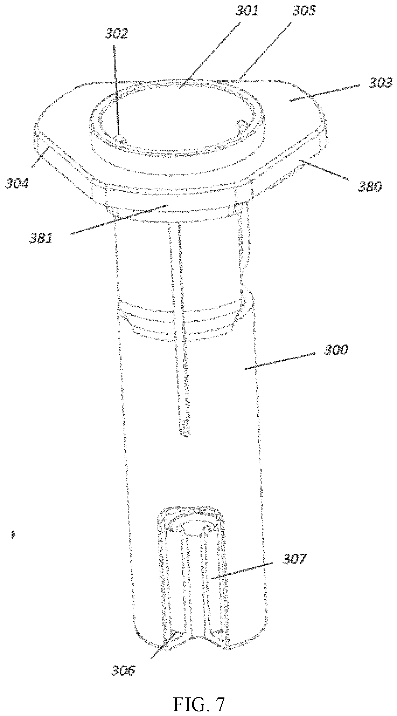

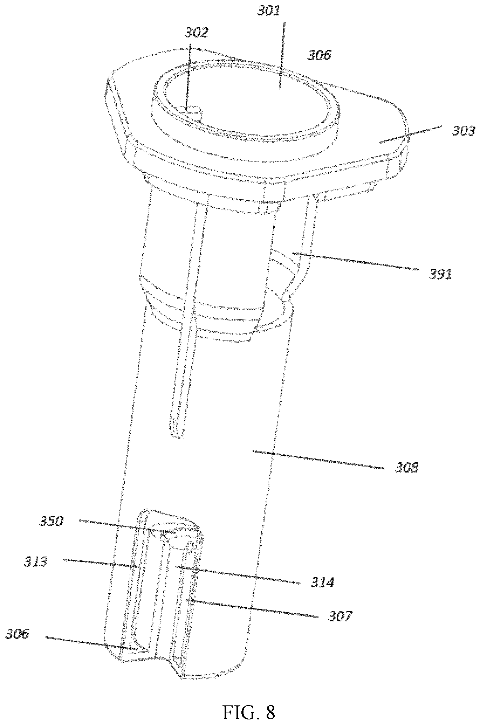

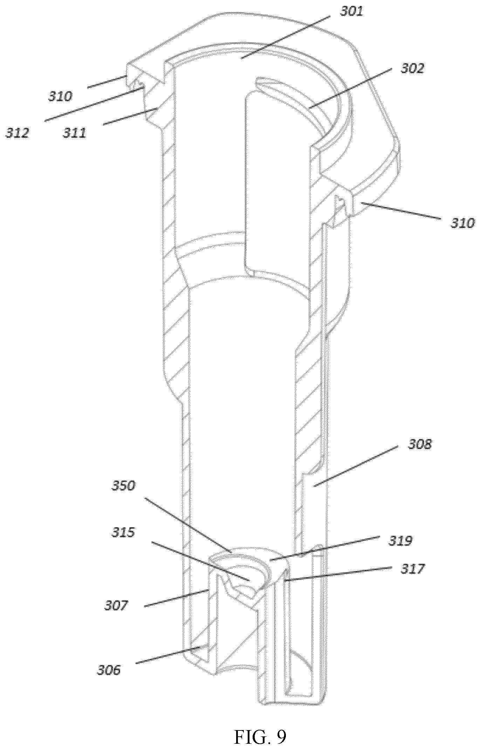

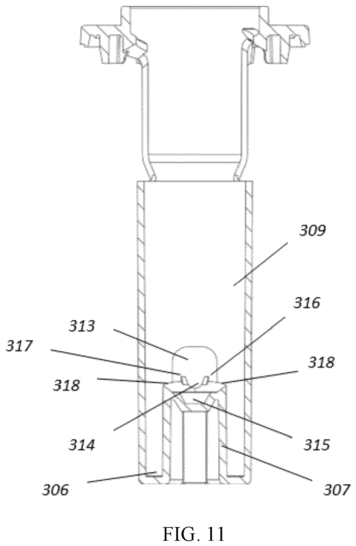

In some embodiments, the invention provides an accommodating chamber or a transfer chamber, and such a chamber is used for receiving the sample chamber and allowing the sample chamber and the accommodating chamber to move relatively, thereby releasing the liquid in the sample chamber into the accommodating chamber. The relative movement here includes the insertion of the sample chamber 400 into the accommodating chamber 300. In some embodiments, as shown in FIG. 7 - FIG. 19B, one accommodating chamber 300 is provided and includes the piercing element. When the sample chamber is inserted into the accommodating chamber, the piercing element contacts with the sealing film at the bottom of the sample chamber, thereby piercing the sealing film and releasing the liquid sample.

In the conventional piercing structure, only a simple piercing element is used for piercing, and the liquid sample is not easy to normally flow out of the sample chamber. For example, the piercing element is provided with a sharp structure such as a needle, so the sealing film can be pierced by an ordinary sharp structure; when the piercing element pierces the sealing film, a part of the piercing element will enter the sample chamber; as the sealing film is generally flexible, even if the piercing element pierces the sealing film, the flexible sealing film will still cover the surface of the piercing element or contact with the piercing element. There is a gap when the piercing element will directly contact with the sealing film, but the liquid is not easy to flow out of the sample chamber. After the piercing element pierces the sealing film, a pierced opening is formed. Although the piercing element departs from the pierced part, the liquid is not easy to normally flow out of the pierced part (pierced opening). This is due to a fact that in normal cases, the amount of the liquid is sometimes very small, and although the sealing film is pierced, the sealing film can recover to its non-piercing state. For example, for some elastic sealing films, the pierced opening is narrowed, and the amount of the liquid is very small (light weight), such that the liquid is not easy to normally flow out by gravity. In the invention, in order to keep the sealing property of the bottom of the sample chamber before pierced, the opening 420 of the sample chamber 400 is sealed, or the partition structure is sealed by the elastic sealing rings 201, 202 on the rod 206 of the sample collector. In fact, the whole lower chamber in which the liquid is accommodated is hermetically sealed or liquid sealed. When the sample chamber 400 is vertically inserted into the accommodating chamber 300, the sealing film is pierced by the piercing element. We find that the liquid is not easy to flow out of the sample chamber, because the sample chamber is sealed, only the pierced part has an opening, and the pierced sealing film can restore an original state. Therefore, even if there is a small opening, the liquid is not easy to naturally flow out or slowly flow out due to the action of air pressure (the action of external atmospheric pressure). If the liquid does not flow out, this will cause the liquid not to flow out or cause the liquid to flow out very little even if the sealing film is pierced, thus causing the failure of the test. Especially, if a small amount of the liquid sample is accommodated in the sample chamber, the liquid sample very slowly flows out normally only by its gravity, and sometimes it does not flow out. Furthermore, when such a sample chamber is used for collecting a tiny amount of the samples, the amount of the treatment liquid in the sample chamber is very small, for example, 50-100 µl, and the sealing film is provided at the bottom of the sample chamber. Even if the sealing film is pierced, the tiny amount of the samples cannot easily flow downward normally by gravity. Furthermore, when the sample chamber of the invention is used for collecting the samples, the collecting head of the sample collector is often immersed in the treatment liquid, such as a water absorption element; filter paper, sponges, cotton swabs, flocked cotton swabs and the like are reserved in the sample chamber alone after the samples are collected, and the sample chamber 400 may be shaken in order to allow the treatment liquid to elute the analyte on the water absorption element. It is also possible that after the samples are collected, they need to be transported to a testing institution for testing instead of being tested immediately. Therefore, the absorbent materials are likely to be dispersed, or some absorbent fibers are dissolved in the treatment liquid, for example, short fibers are suspended in the liquid. Therefore, when the sealing film is pierced by the piercing element, although the pierced opening is present, these dispersed fibers will block the pierced opening or form some dense meshes to cover the pierced opening, and sometimes the liquid will not normally flow out of the sample chamber. In these cases, it is actually difficult to implement piercing only by the conventional piercing element and allow the sample to flow out of the sample chamber smoothly. Moreover, when the piercing element pierces the sealing film without departing from it, the resulting problem is more serious. The piercing structure is still located in the sample chamber, and the pierced opening is blocked by the piercing element. However, a gap that may be formed between the sealing film and the piercing element is very small, and the liquid is not easy to flow out of the sample chamber due to the surface tension. For the ease of operation, it is always hoped that the liquid can be released after the piercing element pierces the sealing film (the piercing element passes through the sealing film and enters the sample chamber). If the piercing element pierces the sealing film and then departs from the pierced film, an additional operation is made, such as controlling the departure of the piercing element or controlling the bottom of the sample chamber to depart from the piercing element. This is cumbersome in operation.

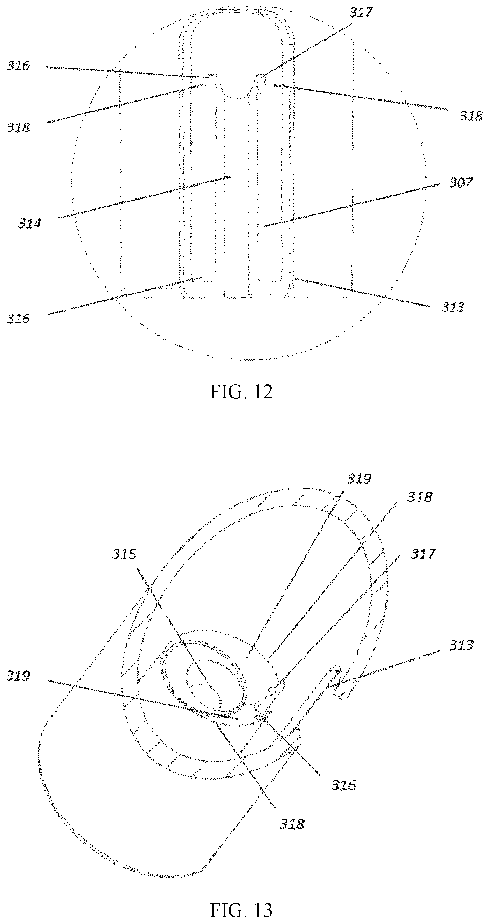

In order to solve the problem encountered by the conventional piercing element and allow the liquid to flow out smoothly or quickly without an additional operation step and a simplified operation process, one of the embodiments provided by the invention is to improve the piercing element, and the piercing element is provided with some blade structures that can tear, cut and shear the sealing film when piercing the sealing film (along the longitudinal direction of the sample chamber), such that other methods can be used for breaking the sealing film during piercing, the sealing film can be opened wider, and sometimes it even falls off in all or in part, for example, laterally tearing or cutting the sealing film. For example, as shown in FIG. 12 - FIG. 16, the piercing element includes sharp protrusions 317, 316, and the sharp protrusions include a sharp structure 390 that is used for piercing the sealing film 404 along the longitudinal direction of the sample chamber. After piercing, the sharp protrusions 317, 316 will pass through the sealing film 404 and enter the bottom of the chamber, and then the blade structure is arranged on both sides of the sharp protrusions 316, 317 to laterally tear, shear and cut the sealing film. In some embodiments, the blade structure includes a blade surface 318 that is similar to the knife edge of scissors or a blade. When the bottom 425 of the sample chamber enters the accommodating chamber, the sealing film 404 at the bottom 425 hits the sharp structure 390 of the piercing element 317 or the piercing element 316, thereby piercing the sealing film to form one pierced opening 493. Then, the sharp structure enters the chamber. In this case, the blade surface 318 distributed on both sides of the sharp structure contacts with the sealing film, the sample chamber is still allowed to move downwards, and the blade surface laterally tears the sealing film at the pierced part (or the pierced opening 493), such that the sealing film is torn off from the bottom 425 in all or in part (as shown in FIG. 16B). Thus, the sealing film 404 at the bottom of the sample chamber can be torn off by applying little pressure to the sample chamber, which makes the liquid easily flow out of the sample chamber. Therefore, in the design of the piercing element, the sharp structure is provided for piercing the sealing film, and then the blade structure for cutting or tearing the sealing film is also provided, where the cutting or tearing can be started from the pierced opening.

The so-called "blade" of the "blade" structure for tearing, cutting and shearing the sealing film of the invention can be understood as the blade of the cutting knife. Here, the blade can be actually configured in a line pattern, namely, a very thin surface, and the edge of the thin surface is in the line pattern, for example, the edge with a thickness of 1 mm or 0.5 mm has the cutting ability, so the so-called blade surface can be used as one surface including the blade and has the cutting ability that is similar to the ability of cutting paper by a knife and scissors. The material of the blade structure here can be any material, such as plastic, metal and alloy. In some embodiments, the piercing element and the blade surface are different structures made of a same material. Cutting, tearing and shearing here are interchangeable. It can be also understood that the sealing film is cut by the sharp blade. Actually, the cutting way features in laterally applying a force to the sealing film, while the piercing way of the piercing element features in piercing the sealing film along the longitudinal axis of the sample chamber. Of course, the rigidity of the blade is greater than that of the sealing film, but this is not a necessary condition. When cutting or tearing is started from the pierced part or the pierced opening, because the opening formed by piercing the sealing film with the sharp structure has some small torn openings instead of being not regular and smooth, the sealing film is easy to crack or cut. If the blade cuts or tears from these small torn openings, it will be much easier, such that the sealing film or a part of the sealing film can be easily torn off from the bottom 425 of the sample chamber 400 or a wider opening can be formed, and then the liquid in the sample chamber 400 is easy to flow out.

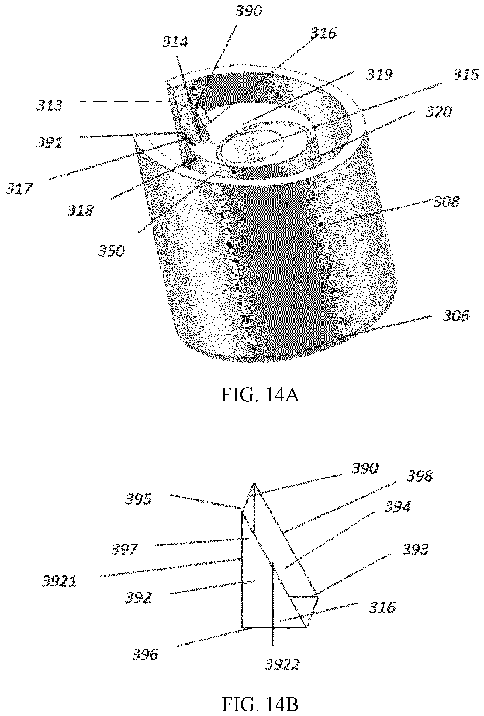

The piercing element can be of any shape, provided that it can pierce the sealing film; and the main function of the blade surface is to cut or tear the sealing film. In some embodiments, the highest apex (sharp structure) of the piercing element is higher than the position of the cutting or tearing blade structure. Therefore, when the piercing element pierces the sealing film having the pierced opening, the cutting or tearing blade surface is cut or torn from the pierced opening, such that the sealing film can be removed from the bottom of the sample chamber in all or in part. For example, as shown in FIG. 14B, the piercing element 316 is similar to an acute triangular cone, including a bottom 393 and a top 390, and a structure enclosed by four faces 392, 394, 395, 398, and the face 395 and the face 394 are obliquely intersected to form an intersection line that forms a sharp piercing structure 390. The piercing structure 390 is a thin and sharp structure and similar to the edge of the thin sheet; the edge is directly opposite to the surface of the sealing film and used for piercing the sealing film, such that the part of the piercing element passes through the sealing film 404 and enters the sample chamber 400. Then, the face formed by sides of the piercing element 316, such as the face 392, can tear the sealing film from the torn opening, the area of the apex of the piercing element is gradually increased from top to bottom, and then the whole piercing element can enter the sample chamber, for example, the whole piercing element from the bottom 393 to the apex 390 enters the sample chamber. The main function of the cutting blade surface is to tear the sealing film from the pierced opening and form a wider pierced opening, and the pierced opening can make the sealing film fall off from the bottom of the sample chamber in all or in part.

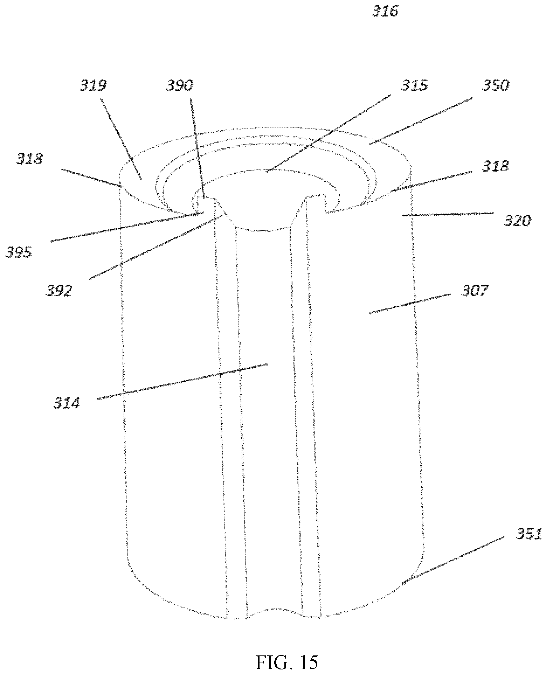

One or more piercing elements here may be provided. The cutting or tearing blade surface may be provided at any position, provided that it contacts with the sealing film after the piercing element contacts with the sealing film. As shown in FIG. 16A - FIG.16D, the sealing film 404 seals the bottom 425 of the sample chamber. Generally, the periphery of the sealing film is bonded to the edge 490 of the bottom of the side wall 403 of the sample chamber, thus sealing the bottom 495 of the sample chamber (FIG. 16A). The position where the piercing element is arranged to pierce the sealing film can also be near the inner wall 199 of the sample chamber 400, for example, the position indicated by the pierced opening 493 or the pierced opening 492, or the position near the inner wall 499 of the sample chamber, in which the sealing film is pierced. The sharp structure 390 of the piercing element is used for piercing the sealing film, thereby forming the pierced opening 493 or the pierced opening 492. Then, the blade structure for cutting or tearing the pierced opening surface is also arranged near the inner wall 499 close to the side wall 403, such that the sealing film can be cut or torn along the pierced opening. As shown in FIG. 16B, the sealing film is torn along the position of a dotted line 494, and then the part of the sealing film 4041 can be torn, cut or sheared from the bottom of the sample chamber (as shown in FIG. 16C), such that the bottom 495 of the whole sample chamber is exposed without the sealing film (as shown in FIG. 16D), and the liquid in the sample chamber 400 can be normally retained. Of course, in this manner, the sealing film can be pierced and cut at any position, and the part of the sealing film can be removed. For example, as shown in FIG. 16B, the sealing film can be pierced at a position 466, and is laterally cut by the blade structure from the pierced opening, and then the part of the sealing film 4042 is cut, thus forming an opening (a blank area) without the sealing film. The shape of the sealing film to be cut can be determined according to the positional arrangement of the piercing element and the cutting blades. As shown in FIG. 15, the sealing film is circular if the piercing element is arranged on the face of the circular blade. When the blade surface 318 is enclosed into other shapes, for example, a square, a circle and an oval; and if the protruding piercing element is arranged on the blade surface 318, the cut sealing film is square, circular or oval. By this way, the sealing film can be cut into any opening. In a preferred embodiment, the sealing film is omitted at the opening, which reduces the interference of the sealing film on liquid flow. That is to say, the piercing element is used for piercing, and the cutting shape of the sealing film is determined by the specific setting pattern of the blade structure.

In the above description, the sample chamber is directly inserted into the accommodating chamber, and the sealing film is cut through the combination of the piercing element and the cutting blade surface , and then the part of the sealing film falls off, which makes the liquid more easily flow out of the sealed sample chamber. In some embodiments, the sample chamber 400 enters the accommodating chamber 300 in a rotation manner, such that the sealing film is more conveniently torn off. In the embodiments, the tearing operation can be implemented by slightly improving the piercing element; for example, the sharp structure 390 is allowed to contact with the sealing film and pierce the sealing film to form one pierced opening. However, when the sample chamber rotates, the sharp piercing element is generally at a fixed position and the sealing film is at a rotary position; after the sharp structure pierces the sealing film, the sealing film is at the rotary position, the sharp structure rotates relative to the sealing film, and the sharp structure directly cuts or tears the sealing film from the pierced part (such as the pierced opening); after the sample chamber rotates by one cycle, the whole sealing film can be cut through the cutting or tearing of the sharp structure; thus, as shown in FIG. 14B, the sharp structure 390 pierces the sealing film to form the pierced opening; the so-called blade structure is formed by the intersection of various faces and used for cutting and tearing. For example, the line 3921 where the face 395 intersects with the face 392 is used as the blade structure, or the line 3922 where the face 394 intersects with the face 392 is used as the blade structure for tearing from the pierced opening. For example, as shown in FIG. 16B, if the piercing structure 390 pierces the sealing film 404 to form the pierced opening 493, the blade surface 3921 or the blade surface 3922 located on the piercing structure cuts or tears the sealing film along the rotation direction of the sample chamber (clockwise-the position indicated by the dotted line 494). Therefore, the blade structure here can be a structure on the piercing element, provided that the structure has a corner angle instead of a curved face. The piercing element here can have two functions. One function is to pierce the sealing film (generally, the piercing element is arranged upright relative to the sealing film, for example, perpendicular to the sealing film). Then, with the rotation of the sample chamber, the piercing element can also have the function of lateral cutting, such that the opening of the sealing film is wider, or the sealing film can be directly removed from the bottom of the sample chamber in all or in part. As shown in FIG. 16B, for example, the sealing film is pierced from the pierced opening 493, the sharp structure passes through the sealing film, the sealing film is at the rotary position and the sharp piercing element 316 is at the fixed position, such that the sharp piercing element laterally tears or cuts the sealing film from the pierced opening 493. As shown in FIG. 16B, the sealing film is torn clockwise. Therefore, the piercing element here not only perpendicularly pierces the sealing film, but also laterally tears the sealing film from the pierced opening. Therefore, in some embodiments, if the sharp shape 390 of the piercing element is used for piercing, and the piercing element has the edge or face of the blade, a lateral action is given to the sealing film. For example, the piercing element 316 has a sharp protrusion 390 for piercing the sealing film, and the line 3921 can give the sealing film a lateral shear force, such that the sealing film can be cut off (FIG. 14B). In some embodiments, the piercing element does not necessarily have the blade surface . As mentioned above, the pierced opening of the sealing film is not a regular opening from the microscopic point of view, but many tiny tear holes are formed in the edge of the opening. If the sealing film moves relative to the piercing element, this is equivalent to that the piercing element laterally moves relative to the sealing film. The piercing element acts on the sealing film laterally, and the sealing film can be torn from the tiny tear holes, thus forming the wider opening and even tearing off the part of the sealing film. When the sealing film is sealed by the aluminum foil or metal sealing film, it can be pierced only by the piercing element, and the wider opening in the sealing film can be easily formed, or the part of the sealing film can be removed from the bottom of the sample chamber, thus eliminating the influence of the sealing film on the flow of the liquid.

In some embodiments, if the blade surface 318 can also be arranged around the piercing element, the sealing film on the rotating sample chamber can be more easily torn off. Thus, the sealing film is at the rotary position and the blade surface 318 is substantially fixed. This is equivalent to that one wider opening in the sealing film is cut by one blade (318), such that the sealing film is more easily removed. In one preferred embodiment, the sealing film is cut or torn from the pierced opening. In some embodiments, the sharp protrusion and the blade surface are arranged near a junction between the sealing film and the side wall of the sample chamber. The sealing film is pierced at the junction and cut off along the junction to make the whole sealing film fall off. When the whole sealing film 404 falls off from the bottom, this is equivalent to that no any sealing film is provided at the bottom, such that the liquid in the sample chamber can more easily flow out.

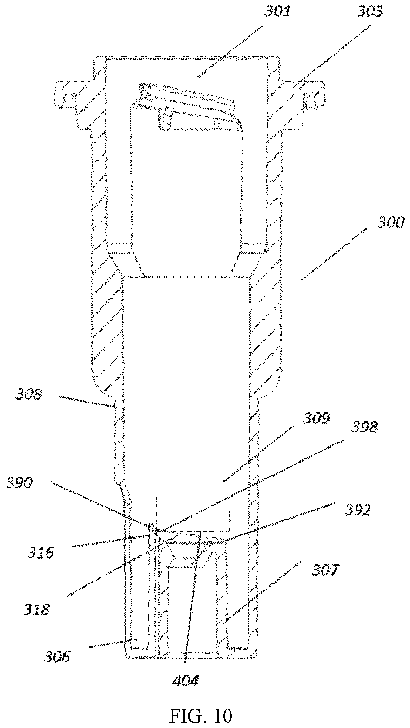

In some embodiments, the blade surface or the blade line is formed in an inclined plane, rather than all the blades being in a same plane. In one embodiment, the position of the blade intersecting the piercing element is higher than that of the blade away from the piercing element. As shown in FIG. 10, the sharp apex 390 of the piercing element is higher than a part of the blade surface 398 adjacent to the piercing element, while a part of the blade surface 392 far away from the piercing element is lower than the blade surface 398 adjacent to the piercing element. Although both the part of the blade surface 398 at a high position and the part of the blade surface 392 at a low position are located on the whole blade surface 318. Thus, when the sealing film moves downward perpendicular to the piercing element, the piercing element perpendicularly pierces the sealing film, thus forming the pierced opening. However, the part of the blade surface 398 is torn or sheared from the pierced opening, and then the part of the blade surface 392 at the low position contacts with the sealing film 404. This is equivalent to that the sealing film is allowed to have a shearing force (the sealing film as shown in FIG. 10 has an included angle with the blade surface ). The blade surface 318 here can be arranged near the piercing elements 317, 316, can be enclosed by the piercing element, or can be arranged in any other forms. For example, as shown in FIG. 14A, the piercing element 317 has the sharp structure 391, the piercing element 316 has the sharp structure 390, and the blade structure 398 is arranged at the bottom of the piercing element and is extended to form an extended blade surface 318. When the blade surface has a weakened face 319, the blade surface 318 is thin and sharp, and the weakened face 319 is concave, such that the blade surface 318 is thinner and sharper. When both the sharp piercing element and the blade surface 318 can be formed by injection molding at one time.

In some embodiments, such a sharp structure or blade surface is provided on a piston 307 and more preferably on the upper surface 350 of the piston. As shown in FIG. 12, FIG. 13 and FIG. 14A, the piston is located at the bottom of the accommodating chamber and protrudes upward from the bottom, and the protruding piercing elements 316, 317 are provided on the periphery 320 of the upper surface 350 of the piston, while the center of the piston is concave and has a collapsed surface 319. A thin surface can be formed on the upper surface of the piston and is the blade surface, the blade or the blade structure 318, where the blade surface surrounds the periphery of the piston and is lower than the protruding piercing elements 317, 316. The piercing element includes the sharp piercing elements 391, 390 and is also very thin sheet (as shown in FIG. 14B), and the piercing element is similar to the acute triangle and arranged upright on the periphery of the upper surface of the piston. In addition, a side (formed by the intersection of adjacent surfaces) 3921 is used as a lateral cutting surface, and gives the sealing film a lateral cutting force when the sample chamber rotates. Thus, when the sample chamber 400 is inserted into the accommodating chamber and descends in the rotation manner, the piercing element 316 pierces the sealing film by the sharp structure 391, and then laterally cuts the sealing film by its cutting edge 3921 (blade surface). In the cutting process, the piston 307 enters the sample chamber. On the one hand, the piston 307 pushes the cut sealing film upward to quickly tear it and allow it to quickly depart from the sealed bottom, thereby making the opening at the bottom wider. On the other hand, the entry of the piston is to give the sealed sample chamber a pressure, such that the liquid flows out of the sample chamber under the pressure (if the chamber for accommodating the treatment liquid is sealed). In order to allow the liquid to flow out of the sample chamber 400 in a relatively fixed direction, in some embodiments, a channel is provided in the piston 307. When the piston enters the sample chamber, a part of the channel also enters the sample chamber 400; and when the liquid needs to flow out of the sample chamber, the liquid flows out along the channel. The liquid flowing out of the channel is tested and assayed. In some embodiments, the piston is provided with two piercing elements 316, 317, and the channel 314 is arranged between the two piercing elements. In some embodiments, the cross-section area of the piston is equivalent to that of the inner section of the bottom of the sample chamber. For example, as shown in FIG. 16B, the cross-section area of the piston is equivalent to that of the sealing film 404, and the shape of the piston is consistent with that of the sealing film, such that both the sharp structures 391, 390 of the piercing element and the blade surface 318 can cut the sealing film along the inner wall 499 of the sample chamber. As the piston enters the sample chamber, the whole sealing film 404 is pushed open or falls off from the bottom of the sample chamber (FIG. 16C). Therefore, the bottom 415 of the whole sample chamber is plugged with the piston, and most of the liquid flows out of the channel. The above improvements can implement that the liquid smoothly and rapidly flow out of the sample chamber under different conditions. Even if fiber impurities are dissolved in the treatment liquid or there are few liquid samples, the sealing film can be completely removed to expose the bottom of the whole sample chamber through the combination of the structural arrangement and operation mode of the piercing element. Under such operation, the liquid sample can flow out of the sample chamber more quickly, thereby reducing the obstruction of the sample chamber on the liquid sample and simplifying operation steps. Neither the piercing element needs to be separated from the sealing film, nor does it need to give a pressure to the sealed sample chamber (such as the lower chamber 405 of the sealed sample chamber 400), so the liquid can normally flow out of the sample chamber by its gravity.

In some embodiments, a way of allowing the rotation of the sample chamber is that the sample chamber rotates in the accommodating chamber through fitting of screw threads provided on the outer wall of the sample chamber 400 with screw threads provided on the inner wall of the opening of the accommodating chamber 300. As shown in FIG. 5A, a threaded surface 406 is arranged outside the sample chamber and provided with threads 402 thereon, and an inner screw thread 302 is arranged on the inner wall of the accommodating chamber 300 near the opening. When the sample chamber 400 is inserted into the accommodating chamber 300, the bottom of the sample chamber is generally allowed to enter the accommodating chamber from the opening 301 of the accommodating chamber, the sample chamber 400 is allowed to move downward in the accommodating chamber 300 in the rotation manner through screw thread fit, and then the piercing element is allowed to pierce the sealing film to form the pierced opening in the sealing film. With the rotation, the piercing element (or the blade surface) is allowed to laterally tear the sealing film, and the cutting blade matching the piercing element can be arranged on the piercing element to give the sealing film a lateral force or can be separately arranged near the piercing element, so as to tear the sealing film from the pierced opening, or cut off a piece from the whole sealing film. As shown in FIG. 16B, the cut sealing film may be C, B or A pieces of the sealing film.

In some embodiments, an opening 313 is formed in the face opposite to the channel 314 in the piston 304 of the accommodating chamber, and faces the channel 314. The opening is exposed out of the channel 314 and can be arranged in the side wall 403 of the accommodating chamber, where the channel is arranged in the piston. Thus, whether the liquid flows out of the channel can be observed through the opening. Of course, the channel can be arranged in the piston instead of the surface of the piston in the form of a groove.

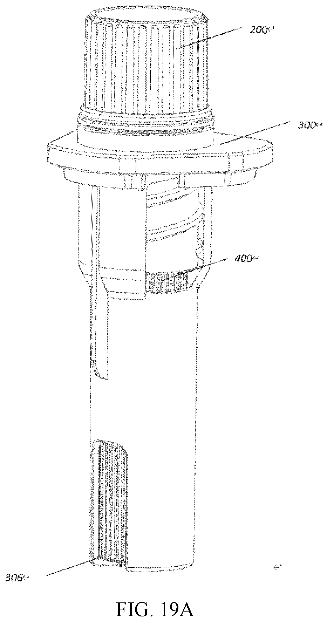

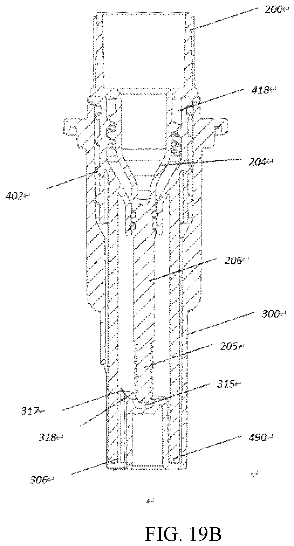

In some embodiments, when the sample is collected by the sample collection area, the sample collection area is located in the lower chamber 405 of the sample chamber and has rigidity. The piston is provided with a recess 315 into which the end of the sample collection area 205 is mainly allowed to be inserted (when the sample chamber is pierced and the piston enters the sample pierced, as shown in FIG. 19), such that the sample chamber 400 can be shorter and miniaturized. Of course, when the brush is used for collecting the sample, the brush can be directly immersed in the treatment liquid in the sample chamber. No matter how long the brush is immersed in the treatment liquid, the liquid can be released smoothly. Through the combination of the piercing element or/and the blade surface of the present piercing and cutting the sealing film and the piston entering the sample chamber in a direction insertion or rotation manner, the above objective can be achieved and some disadvantages of conventional technologies are solved.

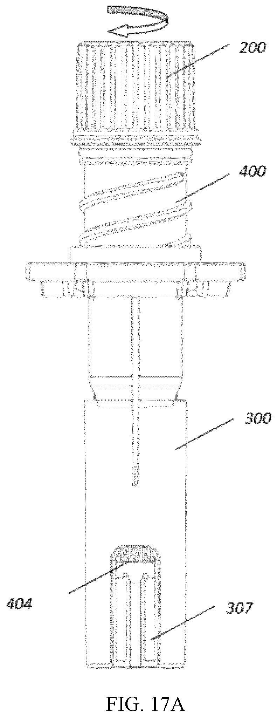

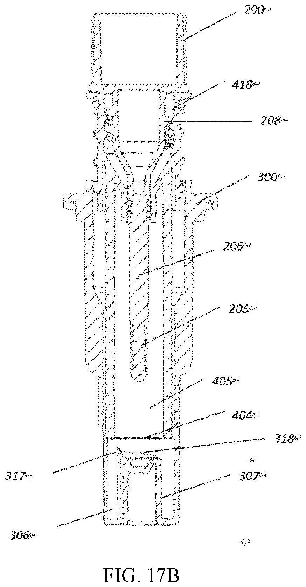

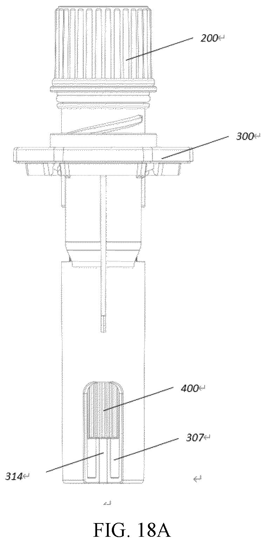

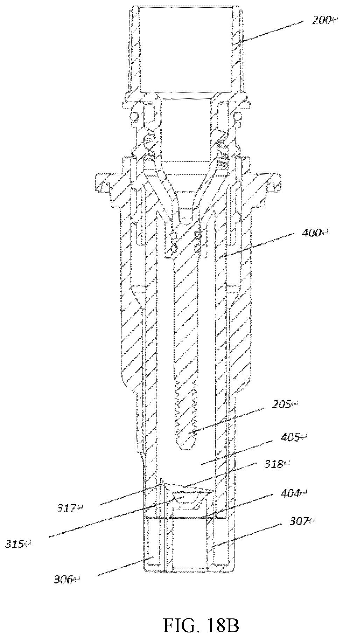

In some embodiments, the specific operation of the rotation is as follows: as shown in FIG. 17A and FIG. 17B, the collector 200 is initially inserted into the sample chamber 400 through the rotation of the screw thread 208 and then clockwise rotated into the sample chamber until it cannot be rotated, such that sealing rings 201, 202 on the collecting rod are sealed with the partition of the sample chamber. Then, the sample chamber 400 with the sample collector is clockwise rotated into the accommodating chamber 300 through the outer screw thread 402 and the inner screw thread 302 at the opening 301 of the accommodating chamber 300. As shown in FIG. 18A and FIG. 18B, with the continuity of the rotation, the piston 307 located on the bottom 306 of the accommodating chamber and the piercing elements 316, 317 arranged on the upper surface of the piston pierce the sealing film to form the pierced opening, and then the pierced opening of the sealing film is laterally cut and torn by the blade surface 318 arranged on the piercing element, such that the opening of the sealing film is enlarged or the part of the sealing film is cut off, and the piston enters the sample chamber to push the torn sealing film open (as shown in FIG. 19A and FIG. 19B), and the liquid in the sample chamber flows out of the channel 314 in the piston.

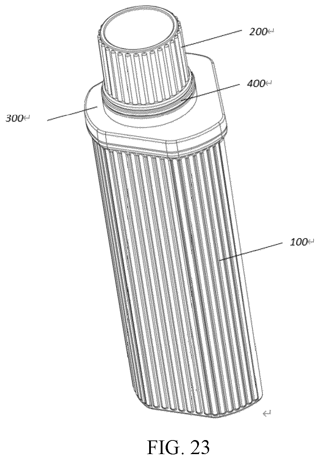

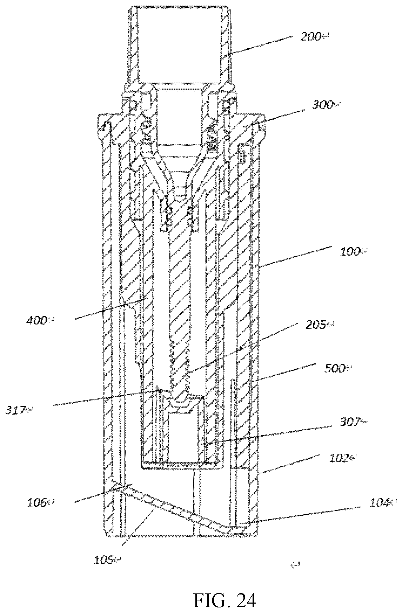

As shown in FIG. 23 and FIG. 24, if the accommodating chamber 300 is assembled in the testing chamber 100, the opening 313 at the bottom of the accommodating chamber faces the side wall 108 of the testing device. If the side walls are transparent, whether the liquid flows out of the channel 314 in the piston 307 can be observed. Similarly, the sample chamber 400 with the sample collector 200 (the sample collector has collected the sample and has been inserted into the sample chamber) enters the accommodating chamber 300 in the testing chamber 100 through screw thread fit, and then the piercing element 316 at the bottom of the accommodating chamber, and then the piercing element with the blade surface 392 or the single blade surface 318 is allowed to pierce the sealing film and cut it laterally, such that the liquid is released onto the inclined plane 108 at the bottom of the testing chamber, flows into the groove 104, and contacts with the sample application area 905 of the test strip arranged here, to assay the analyte. If fecal samples are collected, hemoglobin and helicobacter pylori can be tested. If powder samples are collected, the powder can be tested for presence or absence of one or more substances of drug abuse. If a small amount of saliva samples are collected, the analyte in saliva can be tested, such as small molecules of drug abuse.

### Embodiments

Testing device: when the sample chamber and the sample collector are detachably combined, fecal samples are collected. The lower chamber of the sample chamber 400 is in advance filled with 3 ml treatment liquid (phosphate buffer). The sample collector 200 is initially inserted into the sample chamber. The sample collection area is of a threaded structure 205 which is immersed in the treatment liquid, as shown in FIG. 6. The accommodating chamber is an accommodating chamber as shown in FIG. 7. The piercing element arranged at the bottom 306 of the accommodating chamber is as shown in FIG. 12, FIG. 13 and FIG. 14. A piston 307 is provided at the bottom of the accommodating chamber, and two protruding piercing elements 316, 317 are provided on the peripheral edge of the piston surface, each of which is provided with sharp structures 390, 391. In addition, the blade structure 318 is arranged around the periphery of the piston 307from the bottom of the piercing element (as shown in FIG. 14A and FIG. 14B). The piercing structure and the blade structure are arranged on a surface of the piston 307.

Collection of samples: the fecal samples preserved in the laboratory were used, in which some short fibers (1-5 mm fibers) and hairs were artificially mixed, to simulate some complex fecal samples. Then the sample collector 200 was used for collecting the fecal samples and then inserted to the sample chamber, such that the fecal samples on the screw thread were dissolved in the treatment liquid. After that, the sample collector 200 was inserted into the accommodating chamber 300 through the rotation fit of the inner screw threads and outer screw threads of the sample chamber. In this way, the piercing element at the bottom of the accommodating chamber was allowed to pierce and cut the sealing film, and a volume of liquid sample outflow was observed.

### Embodiment 1: an insertion manner (the threaded structure is omitted in the sample chamber and the accommodating chamber) (fecal samples) is that the sample chamber is directly inserted into the bottom of the accommodating chamber instead of being rotated into the bottom until the sample chamber cannot move.

A total of 100 sample chambers were used for the test and divided into two groups. In one group, the sample chamber and accommodating chamber of the invention were inserted, and a volume of the liquid flowing from the bottom of the sample chamber was recorded. It was found that there were 50 sample chambers, more than 2.5 ml of the liquid flowed out of each of the sample chambers, and most of the sealing films were cut off. Similarly, the other group was used as a control group, where the piercing structure was merely used for piercing, and a piston was also provided. However, there was neither a blade structure on the piercing element, nor a blade surface 318 around the piercing element. The sample chamber entered into the accommodating chamber in an insertion manner instead of a rotation manner. An average volume of the liquid flowing out of the 50 sample chambers was 0.8 ml. Later observation revealed that a small amount of liquid flowed out due to a layer of fibers and hair on the surface of the sealing film of the sample chamber. Although the piercing structure pieced the sealing film, fiber in the treatment liquid blocked the pierced opening, which affected the flow of liquid, and the sealing film had not been cut and was generally maintained on the bottom of the sample chamber. In the above two manners, the piercing element did not depart from the sealing film after piercing it.

### Embodiment 2: a rotation manner (screw thread fit is obtained between the sample chamber and the accommodating chamber) (fecal samples) is that the sample chamber is allowed to enter the accommodating chamber in a thread rotation manner and the bottom of the sample chamber is allowed to move to the bottom of the piston.

A total of 100 sample chambers were used for the test and divided into two groups. In one group, the sample chamber and accommodating chamber of the invention were inserted, and a volume of the liquid flowing from the bottom of the sample chamber was recorded. It was found that there were 50 sample chambers, more than 2.8- 2.9 ml of the liquid flowed out of each of the sample chambers, and all the sealing films were cut off instead of being located at the bottom of the sample chamber, and some of the sealing films were directly jacked by the piston to cover the inner wall of the sample chamber. Similarly, the other group was used as a control group, where the piercing structure was merely used for piercing, and a piston was also provided. However, there was neither a blade structure on the piercing element (the piercing element is sharp, but its main structure is circular), nor a blade structure318 around the piercing element. An average volume of the liquid flowing out of 50 sample chambers was 1.2-1.5 ml, because most of the sealing films were still retained at the bottoms of the sample chambers, reducing the flow of liquid.

### Rotation manner (screw thread fit is obtained between the sample chamber and the accommodating chamber) (a small amount of saliva)

A total of 1 ml of treatment liquid (containing 100 mg of short fibers to simulate a complex solution) was in advance stored in the sample chamber, saliva was taken from the mouth by a sponge swab with a saliva absorption capacity of 50-100 µl, the head of the sponge swab was directly inserted and kept in the treatment liquid in the sample chamber, and then the opening of the sample chamber was sealed by a sealing plug. Such sample chambers were provided in hundred, and divided into two groups, with 50 sample chambers in each group. In one group, the sample chamber and accommodating chamber of the invention were inserted, and a volume of the liquid flowing from the bottom of the sample chamber was recorded. It was found that there were 50 sample chambers, more than 0.8 ml of the liquid flowed out of each of the sample chambers. Similarly, the other group was used as a control group, where the piercing structure was merely used for piercing, and a piston was also provided. However, there was neither a blade structure on the piercing element, nor a blade structure 318 around the piercing element. An average volume of the liquid flowing out of 50 sample chambers was 0.1-0.2 ml. Because the treatment liquid in the sample chamber is small in volume and light in weight, the liquid is more difficult to normally flow out by its own gravity. Through the manner of the invention, the sealing film at the bottom of the sample chamber is removed by shearing, such that the liquid can flow out with almost no resistance. Thus, this can meet the analysis of an analyte in the liquid and separation of multiple different analytes.

All these embodiments as below are also belonging the invention:
1. A device, comprising: a sample chamber for receiving a sample collector, wherein the sample chamber is provided with an easy-to-pierce sealing film; and an accommodating chamber for receiving the sample chamber, wherein the accommodating chamber comprises a piercing element for piercing the sealing film to release a liquid in the sample chamber, and the accommodating chamber comprises a blade structure for laterally cutting the sealing film.
2. The device according to clause 1, wherein the blade structure is arranged on or near the piercing element.
3. The device according to clause 2, wherein when the blade structure is arranged near the piercing element, the blade structure is connected to a piercing structure.
4. The device according to clause 3, wherein when the position of the blade structure is lower than a piercing apex of the piercing element.
5. The device according to clause 2, wherein the blade structure is arranged along a longitudinal direction of the piercing structure.
6. The device according to clause 1, wherein the piercing element is configured to pierce the sealing film in a vertical direction.
7. The device according to clause 1, wherein when the sample chamber has entered the accommodating chamber or enters the accommodating chamber, the sample chamber enters the accommodating chamber in a rotation or rotational manner relative to the accommodating chamber.
8. The device according to clause 7, wherein the accommodating chamber comprises screw threads, the sample chamber comprises screw threads, and the screw threads of the accommodating chamber are allowed to be meshed with the screw threads of the sample chamber, whereby allowing relative rotation of the accommodating chamber and the sample chamber.
9. The device according to clause 8, wherein the accommodating chamber comprises inner screw threads, and the sample chamber comprises outer screw threads.
10. The device according to clause 9, wherein the accommodating chamber is in a stable and fixed state, and the sample chamber enters the accommodating chamber in the rotation or rotational manner.
11. The device according to clause 10, wherein the sealing film is configured to seal a bottom of the sample chamber, and a part of the sealing film is bonded to an edge of a side wall of the sample chamber.
12. The device according to clause 11, wherein the sample chamber comprises an inner wall, and the piercing apex of the piercing element contacts the part of the sealing film near an inner wall of the sample chamber.
13. The device according to clause 1, wherein the accommodating chamber is located in one testing chamber, and the testing chamber comprises an analyte for testing a liquid sample.
14. The device according to clause 13, wherein the sample chamber comprises the collector detachably combined with the sample chamber, the sample chamber is enclosed by an opening, side walls and a bottom with the sealing film, and the opening of the sample chamber is sealed by a part of the collector.
15. The device according to clause 14, wherein the sample chamber has a treatment liquid is accommodated in the sample chamber therein, and the treatment liquid is sealed in the sample chamber.
16. The device according to clause 13, wherein the sample chamber is enclosed by an opening, side walls and a bottom with the sealing film, and the opening of the sample chamber is sealed by a detachable sealing plug.
17. The device according clause 15, further comprising the sample collector, wherein the sample collector is separately arranged outside the sample chamber and not detachably combined with the sample chamber.
18. The device according to clause 16, wherein the sample collector is capable to be used for collecting the liquid sample or a solid powder sample.
19. The device according to clause 1, wherein the accommodating chamber comprises a bottom on which a piston is arranged; and when the piercing element pierces the sealing film, the piston is capable to pass through the sealing film and enter the sample chamber.
20. The device according to clause 19, wherein the piston comprises a face on which both the piercing element and a blade surface are arranged.
21. The device according to clause 19, wherein both the piercing element and the blade surface are arranged on a peripheral edge of the piston.
22. The device according to clause 21, wherein the piercing element is provided in two, and a channel is arranged between the two piercing elements for draining the liquid from the sample chamber.
23. The device according to clause 22, wherein both the piston and the sample chamber are cylindrical, and a diameter of the piston is substantially equal to an inner diameter of the sample chamber.
24. A method for testing an analyte in a liquid sample, comprising: providing a device, wherein the device comprises: a sample chamber for receiving a sample collector, wherein the sample chamber is provided with an easy-to-pierce sealing film; andan accommodating chamber for receiving the sample chamber, wherein the accommodating chamber comprises a piercing element for piercing the sealing film to release the liquid sample in the sample chamber, and the accommodating chamber comprises a blade structure for laterally cutting the sealing film;
   collecting the sample with the sample collector; inserting the sample collector into the sample chamber; allowing the sample chamber to enter the accommodating chamber; allowing the piercing element in the accommodating chamber to pierce the sealing film of the sample chamber to form a pierced opening; and allowing the blade structure to laterally cut and tear the sealing film to release the liquid from the sample chamber.
25. The method according to clause 24, wherein the blade structure is allowed to laterally cut or tear the sealing film from the pierced opening.
26. The device according to clause 25, wherein the blade structure is arranged on or near the piercing element, such that the blade structure on the piercing element is used for tearing or cutting the sealing film while or subsequently after the piercing element is allowed to pierce the sealing film to form the pierced opening.
27. The method according to clause 26, wherein when the blade structure is arranged near the piercing element, the blade structure is connected to a piercing structure, such that the blade structure near the piercing element is used for tearing or cutting the sealing film while or subsequently after the piercing element is allowed to pierce the sealing film to form the pierced opening.
28. The method according to clause 24, wherein the piercing structure is allowed to pierce the sealing film, and then the blade structure is allowed to cut or tear the sealing film.
29. The method according to clause 24, wherein the piercing element is allowed to pierce the sealing film along a longitudinal direction of the sample chamber.
30. The device according to clause 24, wherein the sample chamber is allowed to enter the accommodating chamber in a rotation or rotational manner relative to the accommodating chamber.
31. The device according to clause 30, wherein the accommodating chamber comprises screw threads, the sample chamber comprises screw threads, and the screw threads of the accommodating chamber are allowed to be meshed with the screw threads of the sample chamber, whereby allowing relative rotation of the accommodating chamber and the sample chamber.
32. The method according to clause 31, wherein the accommodating chamber comprises inner screw threads, and the sample chamber comprises outer screw threads.
33. The method according to clause 32, wherein the accommodating chamber is fixed, and the sample chamber is allowed to enter the accommodating chamber in the rotation or rotational manner.
34. The method according to clause 33, wherein the sealing film is configured to seal a bottom of the sample chamber, and a part of the sealing film is bonded to an edge of a side wall of the sample chamber.
35. The method according to clause 34, wherein the sample chamber comprises an inner wall, and a piercing apex of the piercing element is allowed to pierce at a position close to the part of the sealing film near the inner wall of the sample chamber to form the pierced opening.
36. The method according to clause 35, wherein a cutting blade is allowed to cut the part of the sealing film near the inner wall of the sample chamber from the pierced opening, such that the whole sealing film is capable to be cut.
37. The method according to clause 24, wherein the accommodating chamber is fixedly located in one testing chamber, and the testing chamber comprises an analyte for testing a liquid sample.
38. The method according to clause 24, wherein the sample chamber comprises the collector detachably combined with the sample chamber, the sample chamber is enclosed by an opening, side walls and a bottom with the sealing film, and the opening of the sample chamber is sealed by a part of the collector.
39. The method according to clause 24, wherein the sample chamber is enclosed by an opening, side walls and a bottom with the sealing film, and the opening of the sample chamber is sealed by a detachable sealing plug; the sealing plug is removed, the sample collector with the collected sample is inserted into the sample chamber, and then the sample chamber is sealed by the sealing plug.
40. The method according to clause 24, wherein the sample chamber is enclosed by an opening, side walls and a bottom with the sealing film, and the opening of the sample chamber is sealed by a detachable sealing plug; the sealing plug is removed, the sample collector with the collected sample is inserted into the sample chamber, and then the opening of the sample chamber is sealed by the sealing plug.
41. The method according to clause 24, wherein the sample chamber has a treatment liquid therein, and the treatment liquid is sealed in the sample chamber.
42. The method according to clause 41, wherein the device further comprises the sample collector, and the sample collector is separately arranged outside the sample chamber and not detachably combined with the sample chamber.
43. The method according to clause 42, wherein the sample collector is capable to be used for collecting the liquid sample or a solid powder sample and then inserted into the sample chamber; and the solid powder sample is dissolved in the treatment liquid.
44. The method according to clause 24, wherein the accommodating chamber comprises a bottom on which a piston is arranged; and when the piercing element pierces the sealing film, the piston is capable to pass through the sealing film and enter the sample chamber.
45. The method according to clause 44, wherein the piston comprises one face on which both the piercing element and a blade surface are arranged.
46. The method according to clause 45, wherein both the piercing element and the blade surface are arranged on a peripheral edge of the piston.
47. The method according to clause 46, wherein the piercing element is provided in two, and a channel is arranged between the two piercing elements for draining the liquid from the sample chamber.
48. The method according to clause 22, wherein both the piston and the sample chamber are cylindrical, and a diameter of the piston is substantially equal to an inner diameter of the sample chamber.

All the patents and publications mentioned in the description of the invention indicate that these are public technologies in the art and can be used by the invention. All the patents and publications cited here are listed in the references, just as each publication is specifically referenced separately. The invention described herein can be realized in the absence of any one element or multiple elements, one restriction or multiple restrictions, where such restriction is not specifically described here. For example, the terms "comprising", "essentially consisting of" and "consisting of" in each example here may be replaced by the rest 2 terms. The term "alan" here merely means "one", but does not exclude including 2 or more instead of including only one. The terms and expressions which have been employed here are descriptive rather than restrictive, and there is no intention to suggest that these terms and expressions in this description exclude any equivalents, but it is to be understood that any appropriate changes or modifications can be made within the scope of the invention and appended claims. It can be understood that the embodiments described in the invention are some preferred embodiments and features. Any person of ordinary skill in the art can make some modifications and changes according to the spirit of the description of the invention. These modifications and changes are also considered to fall within the scope of the invention and the scope limited by independent claims and dependent claims.

## Claims

1. A test device, comprising:
a sample chamber for receiving a sample collector, wherein the sample chamber is provided with an easy-to-pierce sealing film; and
an accommodating chamber for receiving the sample chamber, wherein the accommodating chamber comprises a piercing element for piercing the sealing film to release a liquid in the sample chamber, and
the accommodating chamber comprises a blade structure for laterally cutting the sealing film;
the sample chamber has a treatment liquid is accommodated in the sample chamber therein, and the treatment liquid is sealed in the sample chamber.

2. The device according to claim 1, wherein the blade structure is arranged on or near the piercing element.

3. The device according to any one of claims 1-2, wherein when the blade structure is arranged near the piercing element, the blade structure is connected to the piercing element.

4. The device according to any one of claims 1-3, wherein the position of the blade structure is lower than a piercing apex of the piercing element.

5. The device according to any one of claims 1-4, wherein when the blade structure is provided on the piercing element, the blade structure is arranged along a longitudinal direction of the piercing element.

6. The device according to any one of claims 1-5, wherein the piercing element is configured to pierce the sealing film in a vertical direction.

7. The device according to any one of claims 1-6, wherein when the sample chamber has entered the accommodating chamber or enters the accommodating chamber, the sample chamber enters the accommodating chamber in a rotation or rotational manner relative to the accommodating chamber.

8. The device according to any one of claims 1-7, wherein the accommodating chamber comprises screw threads, the sample chamber comprises screw threads, and the screw threads of the accommodating chamber are allowed to be meshed with the screw threads of the sample chamber, whereby allowing relative rotation of the accommodating chamber and the sample chamber.

9. The device according to any one of claims 1-8, wherein the sealing film is configured to seal a bottom of the sample chamber, and a part of the sealing film is bonded to an edge of a side wall of the sample chamber.

10. The device according to claim 9, wherein the sample chamber comprises an inner wall, and the piercing apex of the piercing element contacts the part of the sealing film near an inner wall of the sample chamber.

11. The device according to any one of claims 1-10, wherein the accommodating chamber is in a testing chamber, and the testing chamber comprises a test strip for testing an analyte in a sample.

12. The device according to any one of claims 1-11, wherein the test device further comprising a sample collector, wherein the sample collector is separately arranged outside the sample chamber and not detachably combined with the sample chamber.

13. The device according to claim 12, wherein the sample collector is capable to be used for collecting a liquid sample or a solid powder sample.

14. The device according to any one of claims 1-13, wherein the accommodating chamber comprises a bottom on which a piston is arranged; and when the piercing element pierces the sealing film, the piston is capable to pass through the sealing film and enter the sample chamber.

15. The device according to claim 14, wherein the piston comprises a face on which both the piercing element and a blade surface are arranged.

16. The device according to claim 15, wherein both the piercing element and the blade surface are arranged on a peripheral edge of the piston.
